# EUROPEAN PATENT APPLICATION

(11) **EP 4 060 026 A1**
(43) Date of publication of application: **21.09.2022**
(21) Application number: 21163800.2
(22) Date of filing: 19.03.2021
(51) Int. Cl.: C12N 5/0786, C12N 5/0784, A61K 35/15

(54) **EX-VIVO PROLIFERATION OF HUMAN PHAGOCYTIC CELLS**

(71) Applicant: Technische Universität Dresden, 01069 Dresden (DE); Centre National de la Recherche Scientifique (CNRS), 75016 Paris (FR); Université d'Aix-Marseille, 13007 Marseille (FR); INSERM (Institut National de la Santé et de la Recherche Médicale), 75013 Paris (FR)
(72) Inventor: Sieweke, Michael, 01069 Dresden (DE); Elendner, Clara Jana Lui, 01069 Dresden (DE); Favret, Jeremy, 01069 Dresden (DE); Sarrazin, Sandrine, 13007 Marseille (FR)
(74) Representative: Maikowski & Ninnemann Patentanwälte Partnerschaft mbB

(57) **Abstract**

The invention provides human phagocytic cells which are not tumorigenic, yet still capable of cell division ex-vivo, such as in cell culture. A culture containing these cells can be expanded manyfold, that is the cell number in such a culture can be increased by a factor of 10 or more. The cells show characteristics of functional phagocytes in a differentiated state and are capable to alleviate pathological defects in in vivo models.

## Description

### BACKGROUND OF THE INVENTION

The term phagocyte was originally coined to describe cells capable of ingesting foreign material (Metschnikoff, E.; 1884), bacteria and dying or abnormal cells (Metschnikoff, E., 1887), giving rise to the concept of phagocytosis as a defense mechanism to eliminate invading microorganisms ( see Kaufmann, S. H., 2008, for a review of the history).

Phagocytes can respond to both innate and adaptive signals of the immune system to engage in phagocytosis and perform host protective functions targeted to specific pathogens. Phagocytosis also plays an important role in eliminating dying or dead cells, cellular debris and tumor cells. It can also serve tissue homeostatic functions such as surfactant clearance in the lung or synaptic pruning in the brain.

The mononuclear phagocyte system (MPS) represents a subgroup of related phagocytes comprising monocytes, dendritic cells and macrophages (Geissmann F, et al., 2010).

Monocytes circulate in the blood, bone marrow, and spleen in a steady state (Auffray C. et al., 2009; Swirski F.K. et al., 2009).

They represent immune effector cells and can migrate from blood to tissues in response to tissue-derived signals, for example during infection. They produce inflammatory cytokines, take up cells and toxic molecules. Monocytes can also differentiate into DCs or macrophages. Cytokines play an important role in this phenomenon and exposure in vitro to cytokines such as GM-CSF or M-CSF induces differentiation of monocytes toward DCs and macrophages, respectively. Monocytes consist of at least two subsets with different functions and differentiation potential (Auffray C. et al., 2009).

Monocytes are generated in the bone marrow (BM) from granulocyte/macrophage colony forming unit (CFU-GM) progenitors that gives rise to monocytic and granulocytic cells. The maturation process of the monocytic lineage in vivo passes from a monoblast stage, through the promonocyte stage to mature monocytes (Goud TJ et al. 1975). IL-3, GM-CSF and macrophage colony stimulating factor (M-CSF) stimulate in vivo generation of monocytes (Metcalf D., 1990).

In the mouse, monocyte differentiation has been further delineated in great detail and has been shown to pass from hematopoietic stem cells (HSC) through myeloid progenitor stages, which can give rise to all myeloid cells, to the granulocyte-macrophage progenitor (GMP), which gives rise to monocytic and granulocytic cells and may be identical or very similar to CFU-GM progenitors. Yet more immediate monocytic progenitors in the bone marrow are the macrophage/ dendritic cell progenitor (MDP) (Fogg et al. 2006) and lineage restricted monocytic progenitor cell (MoP; Hettinger et al. (2013)).

Newly formed monocytes leave the BM within 24 hours and migrate to the peripheral blood. Circulating monocytes can adhere to endothelial cells of the capillary vessels and are able to migrate into various tissues (van Furth R. 1992), where they can differentiate into macrophages or dendritic cells. Adherence and migration involve surface proteins, lymphocyte-function associated antigen-1 (LFA-1), CD11 and antigen-4 (VLA-4), belonging to the integrin superfamily of adhesion molecules (Kishimoto TK et al. 1989). These integrins interact with selectins on endothelial cells.

Monocyte recruitment to tissues differs under homeostatic and inflammatory conditions and appears to involve two distinct monocyte populations that have been identified in humans and mammalian animal models (Gordon and Taylor, 2005; Auffray C. et al., 2009).

During inflammation monocytopoiesis increases (Shum and Galsworthy, 1982; van Waarde D et al. 1977) resulting in elevated monocyte numbers. Furthermore, inflammatory mediators, IL-1, IL-4, IFN-y and TNF-α upregulate expression of selectins on endothelial cells, promoting migration of monocytes into tissues. The same cytokines modulate expression of integrin adhesion molecules on monocytes (Pober and Cotran, 1990).

At the site of inflammation monocytes are involved in the phagocytosis of opsonized microorganisms or immune complexes via surface Fc γ-receptors (CD64, CD32) and integrin molecules that can serve as complement receptors (CD11b, CD11c). The microorganisms are synergistically killed by reactive oxygen and nitrogen metabolites and through several hydrolytic enzymes (acid phosphatase, esterase, lysozyme and galactosidase) (Kuijpers T. 1989; Hibbs JB et al. 1987). Importantly, monocyte derived macrophages and dendritic cells stimulate T cells by antigen presentation and thus, are involved in the recognition and activation phases of adaptive immune responses (Nathan CF. 1987). Monocytes also secrete a large number of bioactive products which play an important role in inflammatory, proliferative and immune responses, including growth factors (GM-CSF, G-CSF, M-CSF, IL-1) and anti-proliferative factors (IFNs, TNF).

Dendritic cells (DCs) are a distinct lineage of mononuclear phagocytes, specialized in antigen presentation to T cells and the initiation and control of immunity (Steinmann et al., 1974). They are antigen presenting cells with the unique ability to induce a primary immune response (Banchereau and Steinmann, 1998). They can be derived from circulating monocytes or circulating DC progenitors in the blood and non-lymphoid peripheral tissues, where they can become resident cells (Banchereau and Steinmann, 1998; Geissmann, 2007; Wu and Liu, 2007).

Immature DCs (iDCs) recognize pathogens through cell surface receptors, including Toll-like receptors (Reis e Sousa C. 2001). After uptake of antigen DCs mature and migrate to lymph nodes. Mature DCs (mDCs) are efficient antigen presenting cells (APCs) which mediate T cell priming (Banchereau and Steinmann, 1998).

Furthermore, a predominant role of DCs has been described in NK cell activation in mice and humans. Both immature and bacterially activated human monocyte-derived DCs have been shown to induce cytokine secretion and cytotoxicity by NK cells (Ferlazzo G. et al. 2002; Ferlazzo et al. 2003).

Macrophages are resident phagocytic cells present in nearly every tissue of the body. Tissue macrophages serve important roles in the immune response, tissue homeostasis, metabolism and repair. They are involved in steady-state tissue homeostasis via processes such as the clearance of apoptotic cells and the production of growth factors. Macrophage are also equipped with a broad range of pathogen recognition receptors that make them prone to produce inflammatory cytokines and become highly phagocytic during infections (Gordon, 2002). Furthermore, they serve important roles in recruiting or regulating the activity of other lineages of the immune system. Beyond their role in fighting pathogens, macrophages are important in development and tissue repair with infection control being only part of an over-arching role for macrophages in ensuring tissue homeostasis (Nathan, 2008).

Macrophages display enormous functional diversity, responding to tissue-specific signals to engage in tissue specific functions ranging from tissue remodeling to metabolism. They also have central roles in embryonic development that extend throughout life as they contribute to tissue regeneration and remodeling. They are critically involved in wound healing and tissue repair, where they assume trophic functions by removing debris and orchestrating the recruitment and activity of other cell types participating in tissue remodeling (Gordon S, 2003). These roles are also critical under conditions that allow full tissue regeneration (Theret et al., 2019). Macrophages also contribute to energy balance, glucose metabolism and thermoregulation (Nguyen et al., 2011).

Finally, they are key players in tumorigenesis with both pro- and anti-tumorigenic functions depending on their activation and polarization status. This involves phagocytic and cytotoxic functions, modulation of the immune response through release of inflammatory mediators and cytokines, as well as trophic functions directly on tumor cells, on endothelial cells assuring the tumor's energy and oxygen supply and modulation of extracellular matrix composition. Macrophages therefore play important roles in diseases with major significance for public health, such as cancer, cardiovascular, autoimmune, chronic inflammatory, degenerative and metabolic diseases (Qian and Pollard, 2010; Saijo and Glass, 2011; Murray and Wynn, 2011; Chawla et al., 2011; Nahrendorf and Swirski, 2013; Kettenmann et al., 2013; Aguzzi et al., 2013; Wynn et al., 2013).

Macrophages can develop from early primitive macrophage progenitors of the yolk sac before the establishment of definitive hematopoiesis (Ginhoux et al., 2010; Schulz et al., 2012), erythroid-macrophage progenitors (EMP) of diverse hematopoietic sites of the embryo or from embryonic hematopoietic stem cell derived fetal monocytes (Ginhoux and Guilliams, 2016). These embryonic macrophages can persist into adulthood and be maintained long term independently of input from adult hematopoietic stem cells, as shown most prominently for example in the brain, epidermis or lung (Ajami et al., 2007; Chorro et al., 2009; Hashimoto et al., 2013).

Besides this developmental pathway, macrophages can also be derived from blood monocytes after birth. In the steady state the contribution of embryo or monocyte-derived macrophages to distinct tissue macrophage populations can vary largely, for example from nearly excusive embryonic origin of brain microglia to mixed origin in cardiac macrophages and nearly exclusive monocyte origin in intestinal or dermal macrophages. Monocytes can also leave the blood to infiltrate tissues under diverse challenge conditions such as in atherosclerosis (Woollard and Geissmann, 2010), myocardial infarction (Nahrendorf et al., 2010), muscle injury (Arnold et al., 2007) or infection and inflammation (Serbina et al., 2008; Shi and Pamer, 2011).

Infiltration occurs within 1-2 hours or the first 24h, depending on the monocyte subtype (Auffray et al., 2007; Auffray et al., 2009; Nahrendorf et al., 2010; Shi and Pamer, 2011).

Infiltrating monocytes can die in the process, leave the site of inflammation or injury again or contribute to the tissue resident macrophage pool. Such monocyte derived macrophages can show a high degree of heterogeneity that reflects a morphological and functional specification adopted in the infiltrated tissue. The contribution of bone marrow derived cells to macrophages thus is highly tissue specific and differ under homeostatic and inflammatory conditions (Bleriot et al. 2020).

According to their anatomical localization macrophages may also have distinct names (e.g. microglia in the central nervous system, Kupffer cells in the liver).

Irrespective of their initial yolk sac, embryonic or adult origin, tissue macrophage populations can persist long term in tissues and self-maintain with no or minimal input from circulating monocytes, indicating that tissue macrophages require mechanisms for local self-renewal to maintain their numbers in homeostasis and under challenge. The majority of macrophages appear to have only a very limited proliferation capacity (Gordon and Taylor, 2005) with less than 1-2% of proliferating cells in the steady state (Soucie et al., 2016).

Although tissue infiltrating monocytes can have a very limited proliferation ability, monocytes circulating in the blood do not cycle and rapidly differentiate into macrophages rather than expand, when stimulated with M-CSF ex vivo.

The in vitro differentiation of murine macrophages from bone marrow in the presence of M-CSF was described by Stanley et al. (1978, 1986). Whereas progenitor cells will initially proliferate in response to M-CSF, they eventually differentiate to mature macrophages and terminally withdraw from the cell cycle (Pixley and Stanley, 2004; Klappacher et al.,2002). Thus, even though the macrophages generated this way will survive for a limited time, they are not homogeneous and cannot be further expanded in culture. Similarly, human monocytes do not proliferate in response to M-CSF but initiate morphological changes indicative of macrophage differentiation (Becker et al., 1987). Although a significant number of monocytes can be obtained from a patient by leukapheresis and elutriation (Stevenson et al., 1983), these cells will further differentiate to macrophages in a few days without proliferating and cannot be maintained in culture. Finally, various iPS-derived macrophage differentiation protocols give rise to only a limited number of growth-arrested macrophages, with a maximal reported yield of 15 macrophages per input iPS cell (Lee C.Z.W. et al. (2018)).

WO2008/084069 A1 describes a method for expanding monocytes, macrophages or dendritic cells by inhibiting the expression or activity of MafB and c-Maf. Inhibition of gene expression by using siRNA oligonucleotides, antisense oligonucleotides or ribozymes is explicitly mentioned. For mice it is described that mouse embryos deficient for MafB and c-Maf may be obtained by the crossing of MafB-deficient mice with c-Maf deficient mice, and in the experiments MafB, c-Maf double deficient monocytes were obtained from fetal liver cells under conditions that support monocytic growth.

It has been difficult to provide compositions comprising a large number of human non-tumorigenic phagocytic cells, such as macrophages, monocytes or dendritic cells. This is because the human phagocytic cells which have previously been isolated, such as macrophages, monocytes or dendritic cells, had no significant potential, if at all, to proliferate ex-vivo. This meant, for example, that therapeutic approaches which rely on isogenic monocytes or macrophages, were limited to the numbers of cells previously isolated from the patient.

In Nature Biotechnology, Vol.38, May 2020: 509-511, E. Dolgin discusses the advantages and limitations of therapies which are based on CAR-macrophages. While CAR-expressing human macrophages can be generated by very efficient transduction with viral vectors, unlike T-cells (which are easily expanded and on which successful CAR-T anticancer-treatments are based) monocyte-derived human macrophages do not proliferate ex vivo. The dilemma is described as "So, what you put in is basically what you get out." and "That presents a hurdle in terms of the number of cells you have to start with." However, the number of CAR-modified macrophages needed for efficient CAR-M therapy is around 10⁸ to 10⁹ per patient and thus the provision of the cell numbers needed for therapies based on phagocytic cells, such as therapies based on CAR-macrophages, remains a challenge.

It would be desirable to be able to also expand human monocytes, macrophages and dendritic cells. However, in contrast to mice, proliferating human monocytes, macrophages or dendritic cells are only available as tumorigenic cells lines, such as the cell line THP-1, which are not suitable for cell therapies.

### SUMMARY OF THE INVENTION

Now, the present invention provides human phagocytic cells which are not tumorigenic, yet still capable of cell division ex-vivo, such as in cell culture. A culture containing these cells can be expanded manyfold, that is the cell number in such a culture can be increased by a factor of 10 or more. The cells show characteristics of functional phagocytes in a differentiated state and, like fully mature macrophages, they are capable to alleviate pathological defects in in vivo models.

The invention also provides a new ex vivo method for generating, maintaining and expanding human phagocytic cells in long term culture. The inventors have demonstrated that it is possible to expand human phagocytic cells in culture, for example by creating large deletions within all alleles of the MAFB and MAF genes in said cells.

The present invention therefore relates to a human phagocytic cell, wherein the cell is non-tumorigenic and capable to give rise to at least four granddaughter cells under suitable cultivation conditions. The invention also relates to the use of the human phagocytic cells of the invention in medicine, to compositions, such as in particular pharmaceutical compositions, comprising the human phagocytic cells of the invention. An example of the human phagocytic cell of the invention is a cell wherein both alleles of MAFB and both alleles of MAF have been rendered nonfunctional by deletions of at least 50 base pairs. The present inventors have found that such cells have an increased proliferation potential and can be cultured to higher cell numbers than corresponding cells without the deletions. The invention also relates to the use of the human phagocytic cells wherein both alleles of MAFB and both alleles of MAF have been rendered nonfunctional by deletions of at least 50 base pairs in medicine, to compositions, such as in particular pharmaceutical compositions, comprising the human phagocytic cells of the invention. The invention also relates to methods for treating a human subject affected with a disease which can benefit from human phagocytic cell administration, which method comprises administering to said human subject a human phagocytic cell of the invention.

The invention also relates to a composition comprising cells obtainable by cultivation of a collection of human phagocytic cells of the invention. After a significant expansion in cell number, proliferation of the phagocytic cells of the invention in a cell culture may slow down. This collection of cells obtained from the initially proliferating phagocytic cells of the invention can likewise be used in medicine and pharmaceutical compositions can be prepared from them. The invention also relates to a method for generating a human phagocytic cell of the invention, the method comprising effecting two double strand breaks each within both alleles of the MAFB gene and within both alleles of the MAF gene in a human phagocytic cell, and selecting cells with deletions in both alleles of MAFB and in both alleles of MAF. The cells used as starting material for the method can be induced pluripotent stem cells, blood monocytes, cells from cord blood or from bone marrow, to name a few examples.

### Figures

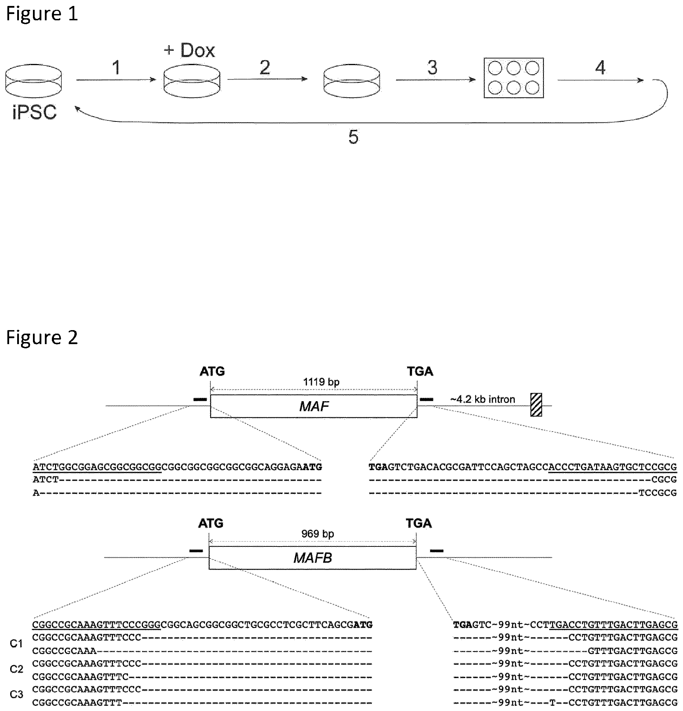

Figure 1: Workflow for CRISPR/Cas9-mediated deletion of MAF and MAFB in a doxycycline-inducible Cas9-expressing human iPSC line
   Step 1: Lipofectamine transfection with a vector expressing MAF-targeting sgRNAs
   Step 2: Cas9 induction upon doxycycline (Dox) treatment
   Step 3: Isolation of reporter-positive, sgRNA expressing cells by cell sorting to derive single-cell colonies
   Step 4: Selection of MAF KO clones
   Step 5: Repeat Steps 1 to 4 using the selected MAF KO iPSC clone with MAFB-targeting sgRNAs to knock out MAFB subsequently
Figure 2: Gene structure of MAF and MAFB indicating position of CRISPR/Cas9 target sites in the 5' and 3' UTR of both genes, and indels obtained.
   The MAF gene has a short and a long isoform generated by alternative splicing, the significance of the long isoform is unknown. We knocked out exon 1 of the MAF gene. MAFB is a single-exon gene. The first line of each sequence block refers to the wild-type locus, subsequent lines the indels generated by CRISPR/Cas9 editing. For MAF, only one clone was isolated (indels on both alleles are shown), for MAFB, the indels for 3 isolated clones are shown (C1, C2, C3: clone 1, 2, 3). In the sequence block, start and stop codons are bold, genomic target sequences are underlined. The protospacer positions are shown as a black line close to the start or stop codon.

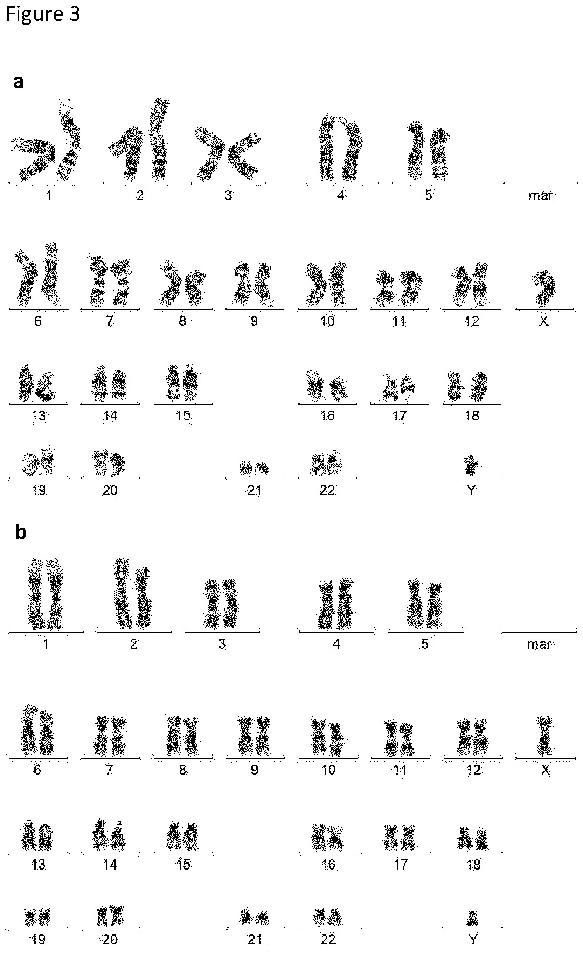

Figure 3: (a) Karyotyping of MAF/MAFB DKO iPSC. (b) Karyotyping of MAF/MAFB DKO iPSC-derived macrophages. DKO-cells at both developmental stages show a normal karyotype.

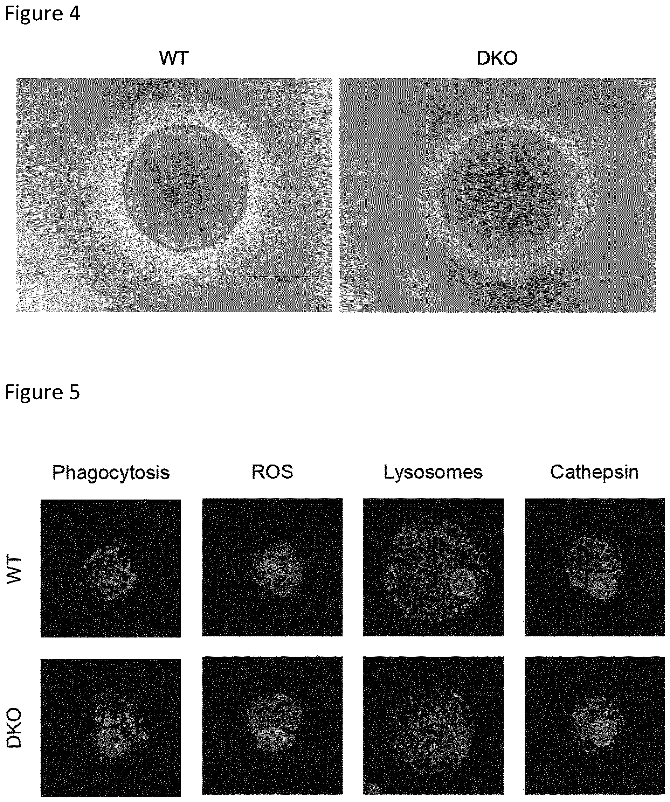

Figure 4: Wild-type and MAF-DKO EBs are morphologically similar. The scale bar represents 300 µm. Images were taken 1 day after EB seeding using 12,500 wild-type or MAF-DKO iPSCs per well of a 96-well plate.
Figure 5: MAF-DKO macrophages are functional macrophages similar to wild-type macrophages. Wild-type and MAF-DKO macrophages were cultured for 7 days in M-CSF and GM-CSF (final differentiation), followed by various functional assays: phagocytosis with Latex beads, staining for reactive oxygen species (ROS) production after overnight stimulation with 100 ng/ml LPS, lysosomal staining with Acridine Orange, cathepsin B activity with the Magic Red kit.

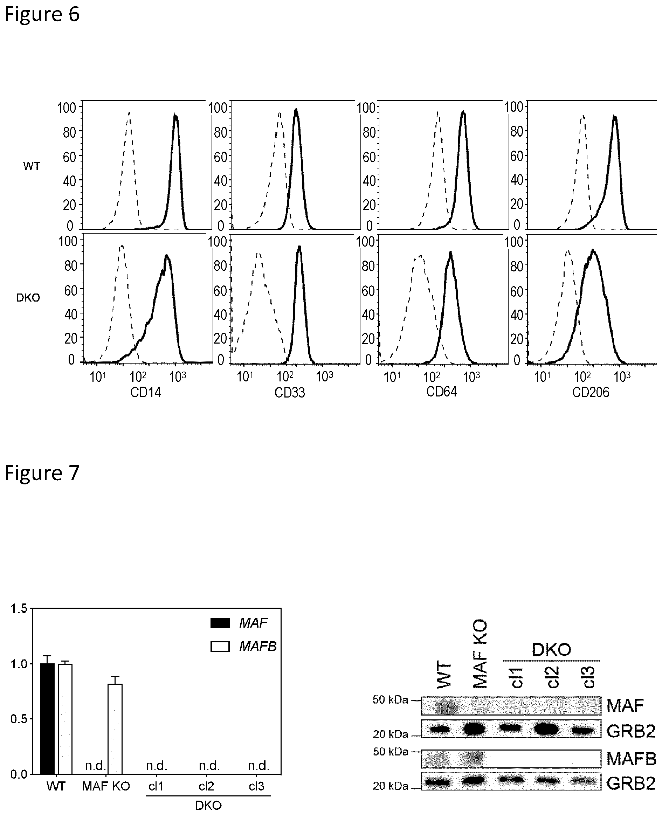

Figure 6: Wild-type and MAF-DKO macrophages express major macrophage markers after culturing 7 days in M-CSF and GM-CSF. Cells were pre-gated for DAPI-negative, CD45+/ CD11b+ cells. Dotted line, FMO (fluorescence minus one) control, black line, stained cells. Wild-type and MAF-DKO macrophages express CD14 (binds LPS), CD33 (myeloid-specific sialoadhesin molecule), CD64 (High affinity Fc receptor binding IgG-type antibodies) and CD206 (mannose receptor) to a similar extent
Figure 7: MAF-DKO macrophages do not express MAF or MAFB. (7 left) RT-qPCR analysis of RNA extracted from wild-type, MAF single KO or three clones of MAF-DKO macrophages. The y axis shows the relative expression of indicated genes (MAF, black; MAFB white) normalized to the control condition, wild-type (WT). (7 right) Western Blot analysis of protein lysate extracted from wild-type, MAF single KO or three clones of MAF-DKO macrophages, WT, wild-type; n.d., not detected; GRB2, loading control.

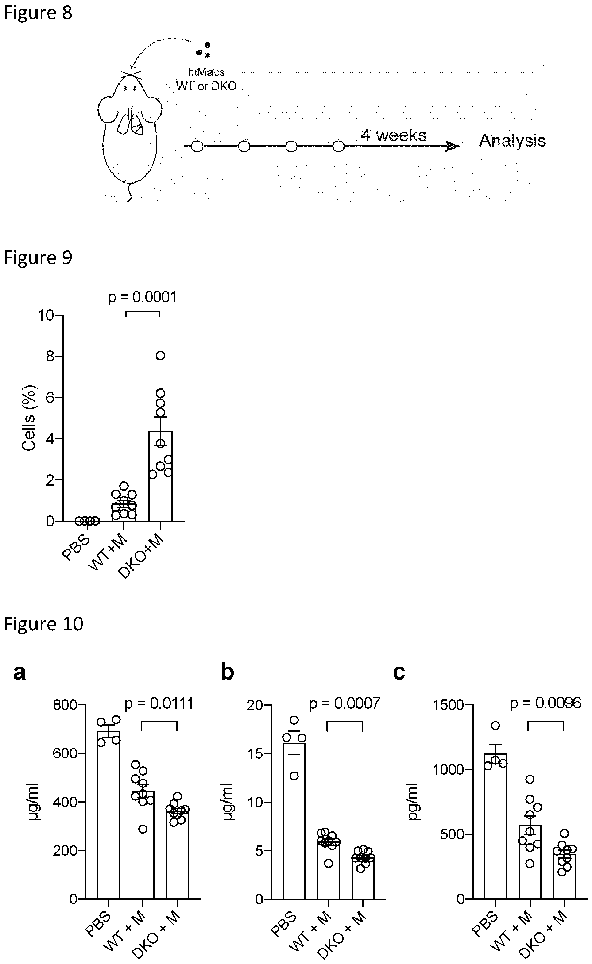

Figure 8: Scheme for in vivo transplantation of wild-type and MAF-DKO macrophages. Equal numbers of wild-type or MAF-DKO macrophages (between 2x10⁶ to 4x10⁶ per administration) were transplanted together with 1µg M-CSF per animal intratracheally 1x/week for 4 weeks. Four weeks after the last transplant, animals were analysed. Each dot on the timeline shows a transplantation, separated by 1 week from the next transplantation.
Figure 9: MAF-DKO macrophages showed better engraftment into huPAP mice than wild-type macrophages. Mice were subjected to bronchoalveolar lavage, cells were isolated by centrifugation, followed by staining for human and mouse CD45, and analysed by flow cytometry. Y axis shows % hCD45+ cells in SSChi (side scatter high) population
Figure 10: MAF-DKO macrophages showed improved rescue of the PAP phenotype compared to wild-type macrophages.
   (a) Protein concentration in BAL fluid measured by BCA assay.
   (b) Concentrations of mouse surfactant protein D (mSPD) in BAL fluid measured by ELISA.
   (c) Concentrations of human GM-CSF in BAL fluid measured by ELISA.

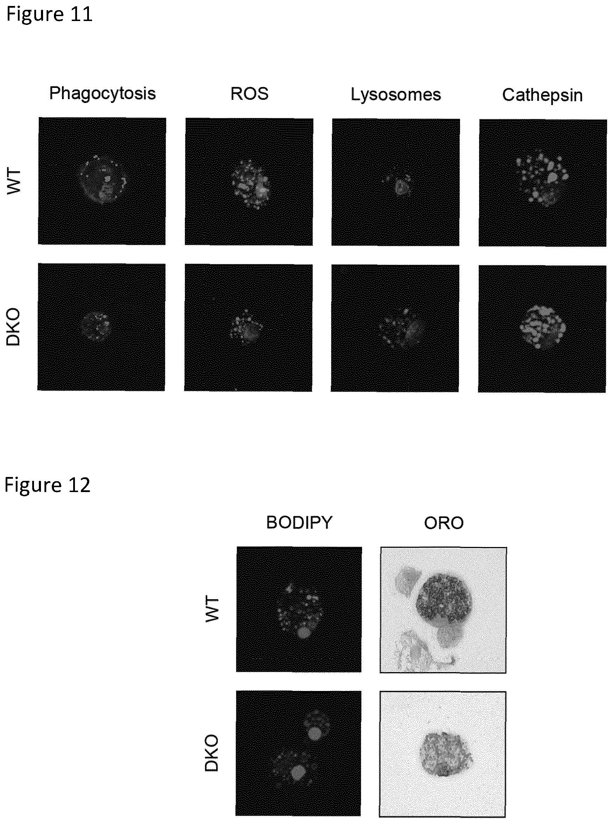

Figure 11: Ex vivo isolated MAF-DKO macrophages are functional macrophages similar to wild-type macrophages. Wild-type and MAF-DKO macrophages were sorted from engrafted huPAP recipients, followed by various functional assays: phagocytosis with Latex beads, staining for reactive oxygen species (ROS) production after overnight stimulation with 100 ng/ml LPS, lysosomal staining with Acridine Orange, cathepsin B activity with the Magic Red kit.
Figure 12: Ex vivo isolated MAF-DKO macrophages can engulf lipids similar to wild-type macrophages. Wild-type and MAF-DKO macrophages were sorted from engrafted huPAP recipients, followed by lipid staining using BODIPY or Oil Red O (ORO).

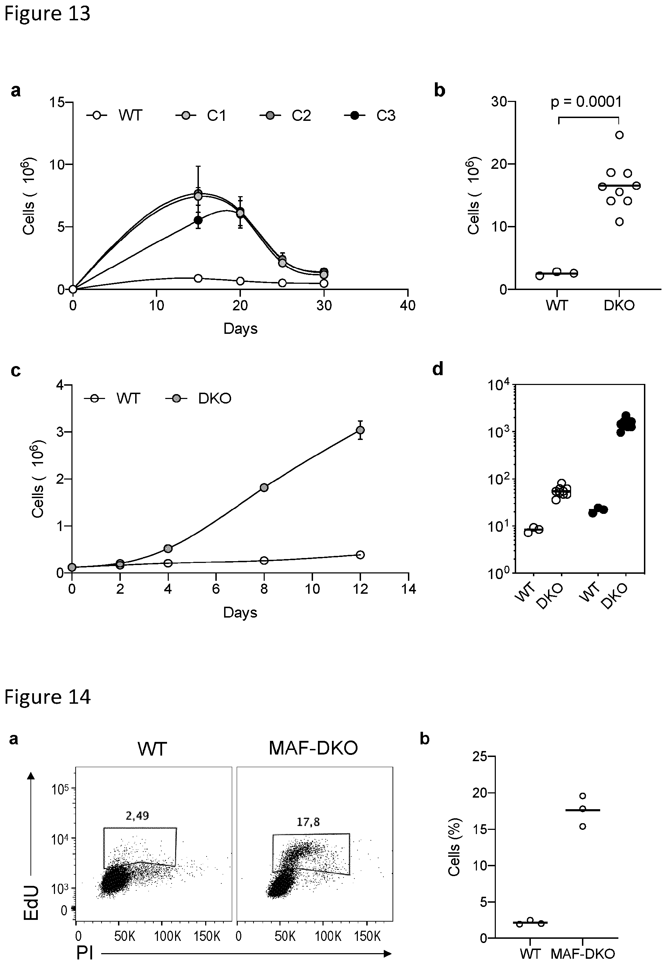

Figure 13: MAF-DKO differentiation results in higher yields than wild-type differentiation.
   (a) Differentiation kinetics of MAF-DKO cells (filled circles) vs. wild-type cells (empty circle) during a period of 30 days. (cells obtained in the supernatant of EB-differentiation cultures)
   (b) Cumulative yield of cells after 30 days of differentiation. Each circle represents one differentiation round, for MAF-DKO all clones were pooled. (sum of all cells harvested from the supernatant of EB-differentiation cultures)
   (c) Final macrophage differentiation of wild-type cells or MAF-DKO cells (clone 1). Day 0 in Fig.13c refers to the day of replating the cells, which had themselves been harvested on day 15 from the EB-differentiation cultures.
   (d) Fold yield normalized to input cell number. Empty circles, yield 30 days after EB-differentiation, but before final macrophage differentiation. Filled circles, yield after final macrophage differentiation
Figure 14: MAF-DKO macrophages incorporate significantly more EdU than wild-type macrophages, indicating a higher proliferative rate. (a) Representative FACS plots showing wild-type and MAF-DKO macrophages after EdU incorporation and PI staining. (b) Quantification of flow cytometric analysis shown in (a). Each dot represents a biological replicate. Y axis, % EdU-positive cells. EdU: 5-ethynyl-2'-deoxyuridine

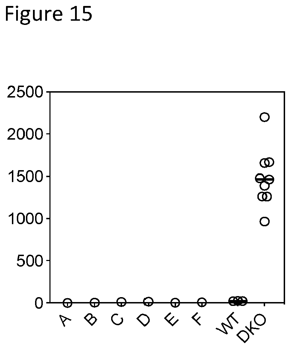

Figure 15: Fold yield calculated as cells obtained after 15 days of EB-differentiation followed by 12 days of final macrophage differentiation, normalized to the cell number used for EB formation (12,500, input cell number). Published values of calculated yields of macrophages obtained per input iPS cells from different differentiation protocols A-D were taken from Lee et al. 2018, E and F values were calculated as number of macrophages obtained per input iPS cell from the data shown in Figure 1A of Buchrieser et al. (2017).

A: Muffat (2016), B: Pandya (2017), C: van Wilgenburg (2013), D: Takata (2017)

### References:

Aguzzi, A., Barres, B. A. & Bennett, M. L. Microglia: scapegoat, saboteur, or something else? Science 339, 156-161, (2013).
Ajami, B., Bennett, J. L., Krieger, C., Tetzlaff, W. & Rossi, F. M. Local self-renewal can sustain CNS microglia maintenance and function throughout adult life. Nat Neurosci 10, 1538-1543 (2007).
Arnold, L. et al. Inflammatory monocytes recruited after skeletal muscle injury switch into antiinflammatory macrophages to support myogenesis. J Exp Med 204, 1057-1069 (2007).
Auffray, C. et al. Monitoring of blood vessels and tissues by a population of monocytes with patrolling behavior. Science 317, 666-670 (2007).
C. Auffray, M. H. Sieweke, F. Geissmann, Annu Rev Immunol 27, 669 (2009).
Aziz A, Soucie E, Sarrazin S, Sieweke MH MafB/c-Maf deficiency enables self-renewal of differentiated functional macrophages Science, 326(5954):867-71 (2009)
Banchereau J, Steinman RM. Dendritic cells and the control of immunity. Nature. 1998; 392:245-252
Ban H, Naoki Nishishita, Noemi Fusaki, Toshiaki Tabata, Koichi Saeki, Masayuki Shikamura, Nozomi Takada, Makoto Inoue, Mamoru Hasegawa, Shin Kawamata, Shin-lchi Nishikawa. Efficient generation of transgene-free human induced pluripotent stem cells (iPSCs) by temperature-sensitive Sendai virus vectors. Proc Natl Acad Sci U S A (2011); 108(34):14234-9
Becker, S., Warren, M.K., and Haskill, S. (1987). Colony-stimulating factor-induced monocyte survival and differentiation into macrophages in serum-free cultures. J Immunol 139, 3703-3709.
Bleriot C., Chakarov S. and Ginhoux F. (2020). "Determinants of Resident Tissue Macrophage Identity and Function" Immunity 52(6):957-970
Buchrieser J., James W. and Moore M.D. (2017) Human Induced Pluripotent Stem Cell-Derived Macrophages Share Ontogeny with MYB-Independent Tissue-Resident Macrophages. Stem Cell Reports 8(2):334-345
Chawla, A., Nguyen, K. D. & Goh, Y. P. Macrophage-mediated inflammation in metabolic disease. Nat Rev Immunol 11, 738-749, (2011).
Chorro, L. et al. Langerhans cell (LC) proliferation mediates neonatal development, homeostasis, and inflammation-associated expansion of the epidermal LC network. J Exp Med 206, 3089-3100, (2009).
Chu, V.T., Graf,R., Wirtz, T., Weber, T., Favret, J., Li, X., Petsch, K., Tran, N.T., Sieweke, M.H., Berek, C., Kühn, R., and Rajewsky, K.: Efficient CRISPR-mediated mutagenesis in primary immune cells using CrispRGold and a C57BL/6 Cas9 transgenic mouse line. PNAS 2016; 113 (44) 12514-12519
E. Dolgin: Nature Biotechnology, Vol.38, May 2020: 509-511,
Ferlazzo G, Morandi B, D'Agostino A, Meazza R, Melioli G, Moretta A, Moretta L. The interaction between NK cells and dendritic cells in bacterial infections results in rapid induction of NK cell activation and in the lysis of uninfected dendritic cells. Eur J Immunol. 2003; 33:306-313
Ferlazzo G, Tsang ML, Moretta L, Melioli G, Steinman RM, Munz C. Human dendritic cells activate resting natural killer (NK) cells and are recognized via the NKp30 receptor by activated NK cells. J Exp Med. 2002;195: 343-351
Fogg DK, Sibon C, Miled C, Jung S, Aucouturier P, Littman DR, Cumano A, Geissmann F. A clonogenic bone marrow progenitor specific for macrophages and dendritic cells. Science. 2006 Jan 6;311(5757):83-7. Epub 2005 Dec 1. Erratum in: Science. 2006 Mar 3;311(5765):1242.
Fusaki, N., Ban, H., Nishiyama, A., Saeki, K. and Hasegawa, M, Efficient induction of transgene-free human pluripotent stem cells using a vector based on Sendai virus, an RNA virus that does not integrate into the host genome. Proc. Jpn. Acad. Ser. B. Phys. Biol. Sci., 85 (2009), p.348-362.
Geissmann, F. (2007). The origin of dendritic cells. Nat Immunol 8, 558-560.
Geissmann F, Manz M, Jung S, Sieweke M, Merad M, Ley K Development of monocytes, macrophages and dendritic cells Science, 327(5966):656-61 (2010)
Ginhoux, F. et al. Fate mapping analysis reveals that adult microglia derive from primitive macrophages. Science 330, 841-845, (2010).
Ginhoux F. and Guilliams M. "Tissue-Resident Macrophage Ontogeny and Homeostasis". Immunity 2016 15;44(3):439-449
Gonzalez F., Zhu, Z., Shi, Z.D., Lelli, K., Verma, N., Li, Q.V., Huangfu, D. An iCRIPR platform for rapid, multiplexable and inducible genome editing in human pluripotent stem cells. Cell Stem Cell (2014) 15, 215-226.
Gordon S, Taylor PR. Monocyte and macrophage heterogeneity. Nat Rev Immunol. 2005 Dec;5(12):953-64
S. Gordon, Cell 111, 927 (Dec 27, 2002).
Gordon S. Alternative activation of macrophages. Nat Rev Immunol. 2003 Jan;3(1):23-35
Goud TJ, Schotte C, van Furth R. Identification and characterization of the monoblast in mononuclear phagocyte colonies grown in vitro. J Exp Med. 1975;142:1180-1199
Graf R, Li X, Chu VT and Rajewsky K. sgRNA Sequence Motifs Blocking Efficient CRISPR/Cas9-Mediated Gene Editing. Cell Reports. 2019 Jan 29
Hashimoto, D. et al. Tissue-Resident Macrophages Self-Maintain Locally throughout Adult Life with Minimal Contribution from Circulating Monocytes. Immunity 38, 792-804, (2013).
Hettinger J., Richards D.M., Hansson J. et al. "Origin of monocytes and macrophages in a committed progenitor". Nat Immunol 2013 Aug;14(8):821-30
Hibbs JB, Jr., Taintor RR, Vavrin Z. Macrophage cytotoxicity: role for Larginine deiminase and imino nitrogen oxidation to nitrite. Science. 1987;235: 473-476
Isogai S, Yamamoto N, Hiramatsu N et al., Cell Reprogram (2018) Dec; 20(6):347-355
Kaufmann, S. H. Immunology's foundation: the 100-year anniversary of the Nobel Prize to Paul Ehrlich and Elie Metchnikoff. Nat Immunol 9, 705-712, (2008).
Karpinski, J., Hauber I., Chemnitz J. et al.: Directed evolution of a recombinase that excises the provirus of most HIV-1 primary isolates with high specificity. Nat. Biotechnol. (2016) 34(4):401-9.
Kettenmann, H., Kirchhoff, F. & Verkhratsky, A. Microglia: new roles for the synaptic stripper. Neuron 77, 10-18, (2013).
Kishimoto TK, Larson RS, Corbi AL, Dustin ML, Staunton DE, Springer TA. The leukocyte integrins. Adv Immunol. 1989;46:149-182
Klappacher, G.W., Lunyak, V.V., Sykes, D.B., Sawka-Verhelle, D., Sage, J., Brard, G., Ngo, S.D., Gangadharan, D., Jacks, T., Kamps, M.P., et al. (2002). An induced Ets repressor complex regulates growth arrest during terminal macrophage differentiation. Cell 109, 169-180.
Kuijpers T. Leukocyte Membrane Adhesion Proteins LFA-1, CR3 and p150,95: a Review of Functional and Regulatory Aspects. Res Immunol. 1989;140: 461-486
Lansing, F., Paszkowski-Rogacz, M., Schmitt, L.T., Schneider, P.M., Romanos, T.R., Sonntag, J., and Buchholz, F.: A heterodimer of evolved designer-recombinases precisely excises a human genomic DNA locus. Nucleic Acids Res. (2020) 48(1):472-485
Lee, C.Z.W., Kozaki T., Ginhoux F. Nat. Rev. Immunol. (2018) 18(11): 716-725.
Liu, H., Zhu, L., Dudiki, T., Gabanic, B., Good, L., Podrez, E. A., Cherepanova, O. A., Qin, J., & Byzova, T. V. (2020). Macrophage Migration and Phagocytosis Are Controlled by Kindlin-3's Link to the Cytoskeleton. The Journal of Immunology, 204(7), 1954-1967. https://doi.org/10.4049/jimmunol.1901134
Metcalf D. The colony stimulating factors: discovery, development, and clinical applications. In: Fortner JG RJ ed. Accomplishments in cancer research. Philadelphia: J.B. Lippincott Company; 1990
Metschnikoff, E. Allg.Wein.med.Ztg 27/29, 307-332 (1884).
Metschnikoff, E. Virchows Archives 107, 209-249 (1887).
Muffat, J. et al. Efficient derivation of microglia-like cells from human pluripotent stem cells. Nat. Med. 22, 1358-1367 (2016).
Murray, P. J. & Wynn, T. A. Protective and pathogenic functions of macrophage subsets. Nat Rev Immunol 11, 723-737, (2011).
Nahrendorf, M. & Swirski, F. K. Monocyte and macrophage heterogeneity in the heart. Circulation research 112, 1624-1633, (2013).
Nahrendorf, M., Pittet, M. J. & Swirski, F. K. Monocytes: protagonists of infarct inflammation and repair after myocardial infarction. Circulation 121, 2437-2445 (2010).
Nathan CF. Secretory products of macrophages. J Clin Invest. 1987;79:319-326
Nathan, C. Metchnikoff's Legacy in 2008. Nat Immunol 9, 695-698,(2008).
Nguyen, K. D. et al. Alternatively activated macrophages produce catecholamines to sustain adaptive thermogenesis. Nature 480, 104-108, (2011).
Pandya, H. et al. Differentiation of human and murine induced pluripotent stem cells to microglia-like cells. Nat. Neurosci. 20, 753-759 (2017).
Pixley FJ, Stanley ER. 2004. CSF-1 regulation of the wandering macrophage: complexity in action. Trends Cell Biol 14: 628-38
Pober JS, Cotran RS. The role of endothelial cells in inflammation. Transplantation. 1990;50: 537-544
Qian, B. Z. & Pollard, J. W. Macrophage diversity enhances tumor progression and metastasis. Cell 141, 39-51, (2010).
Qiu, B., & Simon, M. (2016). BODIPY 493/503 Staining of Neutral Lipid Droplets for Microscopy and Quantification by Flow Cytometry. BIO-PROTOCOL, 6(17).https://doi.org/10.21769/BioProtoc.1912
Rauschmeier, R., Gustafsson, C., Reinhardt, A., A-Gonzalez, N., Tortola, L., Cansever, D., Subramanian, S., Taneja, R., Rossner, M. J., Sieweke, M. H., Greter, M., Månsson, R., Busslinger, M., & Kreslavsky, T. (2019). Bhlhe40 and Bhlhe41 transcription factors regulate alveolar macrophage self-renewal and identity. The EMBO Journal, 38(19). https://doi.org/10.15252/embj.2018101233
Reis e Sousa C. Dendritic cells as sensors of infection. Immunity. 2001;14: 495-498
Saijo, K. & Glass, C. K. Microglial cell origin and phenotypes in health and disease. Nat Rev Immunol 11, 775-787, (2011).
Scharenberg, S.G., Poletto, E., Lucot, K.L., Colella, P., Sheikali, A., Montine, T.J., Porteus, M.H., and Gomez-Ospina, N.: Engineering monocyte/macrophage-specific glucocerebrosidase expression in human hematopoietic stem cells using genome editing. Nat. Commun. (2020) 11(1):3327.
Schulz, C. et al. A lineage of myeloid cells independent of Myb and hematopoietic stem cells. Science 336, 86-90, (2012).
Serbina, N. V., Jia, T., Hohl, T. M. & Pamer, E. G. Monocyte-mediated defense against microbial pathogens. Annu Rev Immunol 26, 421-452 (2008).
Shi, C. & Pamer, E. G. Monocyte recruitment during infection and inflammation. Nat Rev Immunol 11, 762-774, (2011).
Shi, J., Hua, L., Harmer, D., Li, P., Ren, G.: Cre Driver Mice Targeting Macrophages. Methods Mol Biol. (2018) 1784:263-275.
Shum DT, Galsworthy SB. Stimulation of monocyte production by an endogenous mediator induced by a component from Listeria monocytogenes. Immunology. 1982;46:343-351
Erinn L. Soucie, Ziming Weng, Laufey Geirsdóttir, Kaaweh Molawie, Julien Maurizio, Romain Fenouil, Noushine Mossadegh-Keller, Gregory Gimenez, Laurent VanHille, Meryam Beniazza, Jeremy Favret, Carole Berruyer, Pierre Perrin, Nir Hacohe, J.-C. Andrau, Pierre Ferrier, Patrice Dubreuil, Arend Sidow, Michael H. Sieweke Activation of Self-Renewal Gene Network on a Macrophage-Specific Enhancer Platform Science, 351(6274):aad5510 (2016)
Stanley, E.R., Chen, D.M., and Lin, H.S. (1978). Induction of macrophage production and proliferation by a purified colony stimulating factor. Nature 274, 168-170.
Stanley, E.R. (1986). Action of the colony-stimulating factor, CSF-1. Ciba Found Symp 118, 29-41.
Stevenson, H.C., Miller, P., Akiyama, Y., Favilla, T., Beman, J.A., Herberman, R., Stull, H., Thurman, G., Maluish, A., and Oldham, R. (1983). A system for obtaining large numbers of cryopreserved human monocytes purified by leukapheresis and counter-current centrifugation elutriation (CCE). J Immunol Methods 62, 353-363.
R. M. Steinman, D. S. Lustig, Z. A. Cohn, J Exp Med 139, 1431 (Jun 1, 1974).
F. K. Swirski et al., Science 325, 612 (Jul 31, 2009).
Takata, K. et al. Induced-pluripotent-stem-cell-derived primitive macrophages provide a platform for modeling tissue-resident macrophage differentiation and function. Immunity 47, 183-198.e6 (2017).
Thomas, J. R., Appios, A., Zhao, X., Dutkiewicz, R., Donde, M., Lee, C. Y. C., Naidu, P., Lee, C., Cerveira, J., Liu, B., Ginhoux, F., Burton, G., Hamilton, R. S., Moffett, A., Sharkey, A., & McGovern, N. (2020). Phenotypic and functional characterization of first-trimester human placental macrophages, Hofbauer cells. Journal of Experimental Medicine, 218(e20200891).https://doi.org/10.1084/jem.20200891
Theret M, Mounier R, Rossi F. Development. 2019 May 2;146(9)
van Furth R. Production and Migration of Monocytes and Kinetics of Macrophages. In: van Furth R ed. Mononuclear Phagocytes. Dordrecht, The Netherlands: Kluwer Academic Publishers; 1992
van Waarde D, Hulsing-Hesselink E, Sandkuyl LA, van Furth R. Humoral regulation of monocytopoiesis during the early phase of an inflammatory reaction caused by particulate substances. Blood. 1977;50:141-154
van Wilgenburg, B., Browne, C., Vowles, J. & Cowley, S. A. Efficient, long term production of monocyte-derived macrophages from human pluripotent stem cells under partly-defined and fully-defined conditions. PLOS ONE 8, e71098 (2013).
Wang, B., Zuo, J., Kang, W., Wei, Q., Li, J., Wang, C., Liu, Z., Lu, Y., Zhuang, Y., Dang, B., Liu, Q., Kang, W., and Sun, Y.: Generation of Hutat2: Fc Knockin Primary Human Monocytes Using CRISPR/Cas9. Mol Ther Nucleic Acids (2018) 11:130-141
Woollard, K. J. & Geissmann, F. Monocytes in atherosclerosis: subsets and functions. Nat Rev Cardiol 7, 77-86, (2010).
Wu, L., and Liu, Y.J. (2007). Development of dendritic-cell lineages. Immunity 26, 741-750.
Wynn, T. A., Chawla, A. & Pollard, J. W. Macrophage biology in development, homeostasis and disease. Nature 496, 445-455, (2013).
Yumlu S., Stummac J., Bashir S., Dreyer A.K., Lisowski P., Danner E., Kühn R. Methods Vol. 121-122, (2017), 29-44
Zhang, S., Shrestha, C.L., Wisniewski, B.L., Pham, H., Hou, X., Li, W., Dong, Y., Kopp, B.T.; Consequences of CRISPR-Cas9-Mediated CFTR Knockout in Human Macrophages. Front. Immunol. (2020) 11:1871

### DETAILED DESCRIPTION OF THE INVENTION

### Definitions:

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for a particular sequence of amino acids which comprise all or part of one or more proteins or enzymes, and may or may not include regulatory DNA sequences, such as promoter sequences, which determine for example the conditions under which the gene is expressed. A "promoter" or "promoter sequence" is a DNA regulatory region capable of binding RNA polymerase in a cell and initiating transcription of a downstream (3' direction) coding sequence. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

As used herein, references to specific proteins (e.g., the mouse proteins MafB or c-Maf) can include a polypeptide having a native amino acid sequence, as well as variants and modified forms regardless of their origin or mode of preparation. A protein that has a native amino acid sequence is a protein having the same amino acid sequence as obtained from nature (e.g., a naturally occurring MafB or c-Maf). Such native sequence proteins can be isolated from nature or can be prepared using standard recombinant and/or synthetic methods. Native sequence proteins specifically encompass naturally occurring truncated or soluble forms, naturally occurring variant forms (e.g., alternatively spliced forms), naturally occurring allelic variants and forms including postranslational modifications. A native sequence protein includes proteins following post-translational modifications such as glycosylation, or phosphorylation, ubiquitination, sumoylation or other modifications of some amino acid residues.

The terms "mutant" and "mutation" mean any detectable change in genetic material, e.g. DNA, RNA, cDNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. Mutations include deletion, insertion or substitution of one or more nucleotides. The mutation may occur in the coding region of a gene (i.e. in exons), in introns, or in the regulatory regions (e.g. enhancers, response elements, suppressors, signal sequences, polyadenylation sequences, promoters) of the gene. Generally, a mutation is identified in a subject by comparing the sequence of a nucleic acid or polypeptide expressed by said subject with the corresponding nucleic acid or polypeptide expressed in a control population. Where the mutation is within the gene coding sequence, the mutation may be a "missense" mutation, where it replaces one amino acid with another in the gene product, or a "nonsense" mutation, where it replaces an amino acid codon with a stop codon. A mutation may also occur in a splicing site where it creates or destroys signals for exon-intron splicing and thereby lead to a gene product of altered structure. A mutation in the genetic material may also be "silent", i.e. the mutation does not result in an alteration of the amino acid sequence of the expression product.

As used herein the term "expression" may refer to expression of a polypeptide or protein, or to expression of a polynucleotide or gene, depending on the context. Expression of a polynucleotide may be determined, for example, by measuring the production of RNA transcript levels using methods well known to those skilled in the art. Expression of a protein or polypeptide may be determined, for example, by immunoassay using an antibody(ies) that bind with the polypeptide using methods well known to those skilled in the art.

An "inhibitor of expression" refers to a natural or synthetic compound that reduces or suppresses the expression of a gene.

An "inhibitor of activity" has its general meaning in the art, and refers to a compound (natural or not) which has the capability of reducing or suppressing the activity of a protein.

The term "MAF" denotes the human MAF proto-onocogene. MAF and other Maf family members form homodimers and heterodimers with each other and with Fos and Jun, consistent with the known ability of the AP-1 proteins to pair with each other (Kerppola, T. K. and Curran, T. (1994) Oncogene 9:675-684; Kataoka, K. et al. (1994) Mol. Cell. Biol. 14:700-712). The DNA target sequence to which MAF homodimers bind, termed the MAF response element (MARE), is a 13 or 14 bp element which contains a core TRE (T-MARE) or CRE (C-MARE) palindrome respectively, but MAF may also bind to DNA sequences diverging from these consensus sites including composite AP-1/MARE sites and MARE half sites with 5' AT rich extensions (Yoshida et al., NAR2005). MAF has been shown to stimulate transcription from several promoters, as well as to repress the transcription of other promoters. MAF has also been shown to induce the differentiation of T helper 2 (Th2) cells (Ho et al., 1996) due to its ability to activate the tissue specific transcription of the interleukin-4 (IL-4) (Kim et al. 1999). Furthermore, the over-expression of MAF in myeloid cell lines induces macrophage differentiation (Hegde et al., 1999). The gene for human MAF is located on chromosome 16, location 16q23.2 and is described in detail as Gene ID 4094 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for MAF on the complementary strand between 79,593,838 and 79,600,737.

The term "MAFB" denotes the human MAFB transcription factor. This gene is expressed in a variety of cell types (including lens epithelial, pancreas endocrine, epidermis, chondrocyte, neuronal and hematopoietic cells) and encodes a protein containing a typical bZip motif in its carboxy-terminal region. In the bZip domain, MAFB shares extensive homology not only with MAF but also with other Maf-related proteins. MAFB can form a homodimer through its leucine repeat structure and specifically binds Maf-recognition elements (MAREs) palindromes, composite AP-1/MARE sites or MARE halfsites with AT rich 5' extensions (Yoshida, et al. 2005). In addition, MAFB can form heterodimers with c-/v-Maf or Fos through its zipper structure but not with Jun or other Maf family members (Kataoka et al., 1994). MAFB is also known under the name kreisler, kr or Krml1 (for 'Kreisler Maf leucine Zipper 1'), because an x-ray induced chromosomic micro-inversion in kreisler mutant mice causes the tissue specific loss of MAFB expression in the developing hindbrain that is responsible for the kreisler phenotype (Cordes et al., 1994) (Eichmann et al., 1997). In the hematopoietic system MAFB is expressed selectively in the myeloid lineage and is up-regulated successively during myeloid differentiation from multipotent progenitors to macrophages. Indeed, this induction reflects an important role of MAFB in monocytic and macrophage differentiation. Thus the overexpression of MAFB in transformed chicken myeloblasts (Kelly et al., 2000, Bakri et al.2005) and in human hematopoetic progenitors (Gemelli et al., 2006) inhibits progenitor proliferation (Tillmanns et al., 2007) and stimulates the rapid formation of macrophages (Kelly et al., 2000, Bakri et al.2005, Gemelli et al., 2006), whereas a dominant negative version of MAFB inhibits this process (Kelly et al., 2000), indicating that MAFB induction is a specific and important determinant of the monocytic program in hematopoietic cells.

The gene for human MAFB is located on chromosome 20, location 20q12 and is described in detail as Gene ID 9935 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for MAFB on the complementary strand between 40685848 and 40689236.

HLA-A or "human leukocyte antigen 1" relates to a protein belonging to the HLA class I heavy chain paralogues. This class I molecule is a heterodimer consisting of a heavy chain and a light chain (beta-2 microglobulin). The heavy chain is anchored in the membrane. Class I molecules play a central role in the immune system by presenting peptides derived from the endoplasmic reticulum lumen so that they can be recognized by cytotoxic T cells. They are expressed in nearly all cells. The heavy chain is approximately 45 kDa and its gene contains 8 exons. Exon 1 encodes the leader peptide, exons 2 and 3 encode the alpha1 and alpha2 domains, which both bind the peptide, exon 4 encodes the alpha3 domain, exon 5 encodes the transmembrane region, and exons 6 and 7 encode the cytoplasmic tail. Polymorphisms within exon 2 and exon 3 are responsible for the peptide binding specificity of each class one molecule. Typing for these polymorphisms is routinely done for bone marrow and kidney transplantation. More than 6000 HLA-A alleles have been described. The gene for human HLA-A is located on chromosome 6, location 6p22.1 and is described in detail as Gene ID 3105 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for HLA-A on the coding strand between 29942532 and 29945870.

B2M is a protein found in association with the major histocompatibility complex class I heavy chain. The gene for human B2M is located on chromosome 15, location 15q21.1 and is described in detail as Gene ID 567 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for B2M on the coding strand between 44711517 and 44718145.

A "site specific endonuclease" as used herein is an enzyme that cleaves a phosphodiester bond within a polynucleotide chain only at a very specific nucleotide sequence in the middle (endo) portion of a double-stranded DNA molecule, which sequence occurs preferably only once within the whole human genome so as to allow specific genetic engineering of a human target cell. Examples of site-specific endonucleases, which are typically used for genetic engineering in human target cells are zinc finger nucleases, TALENs and the CRISPR/Cas9 system.

"Mutangenesis" as used herein is a laboratory process by which the genetic information of an organism is deliberately changed, resulting in a mutation. Preferred methods for mutagenesis herein are transposon mutagenesis and signature tagged mutagenesis.

The term "proliferating cell" as used herein refers to a cell that is capable of cell division. A cell is a proliferating cell if a population of at least 1000 "proliferating cells" increases in cell number by at least 4-fold after 8 days under suitable cultivation conditions, i.e. when n(192h) / n(0h) is at least 4,00 with n being the total number of cells in the cell population at the indicated time points.

The term "differentiated human cell" as used herein is a cell that does not change cell type and even upon cell division gives rise to two cells of the same cell type. This is in contrast to "pluripotent cells" which can differentiate into all cell types of the adult organism and oligopotent cells, which can differentiate into a few closely related cell types.

An "allele" as used herein refers to one of the two copies of the same human gene. The two copies of a gene, one each on the two homologous chromosomes, can be identical or vary slightly in their individual sequences. The term allele is thus used slightly differently from its typical use herein, because it includes identical versions of the same human gene on the same relative place on the two homologous chromosomes. In a diploid cell, the two alleles of a given gene occupy corresponding loci on a pair of homologous chromosomes.

As used herein an "allele" or a "gene" is rendered nonfunctional if no further expression of the protein encoded by the gene or allele is detectable after the procedure that rendered the allele or gene nonfunctional. That is, no new protein is being expressed from an allele or gene that has been rendered nonfunctional. Eventually the protein encoded by an allele or gene that has been rendered nonfunctional will become undetectable in a population of cells consisting of cells with only nonfunctional alleles. The time until the protein encoded by said gene or allele will become undetectable depends on the dynamics of protein and mRNA turnover for said gene.

As used herein the term "deletion" means that a part of a DNA-sequence is missing compared to a reference sequence.

As used herein an "exon" is any part of a gene that will encode a part of the final mature RNA produced by that gene after introns have been removed by RNA splicing. The term exon refers to both the DNA sequence within a gene and to the corresponding sequence in RNA transcripts. In RNA splicing, introns are removed and exons are covalently joined to one another as part of generating the mature messenger RNA.

A "myeloid cell" as used herein is a cell of hematopoietic origin that is not lymphoid and not erythro-megakaryocytic and not a multi-lineage progenitor with more than myeloid lineage potential.

An iPS cell or "induced pluripotent stem cell" as used herein means a cell having pluripotency, which is obtained by reprogramming a somatic cell. Several groups including the group of Professor Shinya Yamanaka et al. of Kyoto University, the group of Rudolf Jaenisch et al. of Massachusetts Institute of Technology, the group of James Thomson et al. of the University of Wisconsin, and the group of Konrad Hochedlinger et al. of Harvard University have succeeded in producing such induced pluripotent stem cells. The induced pluripotent stem cells have attracted great interest as ideal pluripotent cells having neither immunological rejections nor ethical issues. For example, International Publication WO2007/069666 describes nuclear reprogramming factors for somatic cells that include the gene products of Oct family gene, Klf family gene and Myc family gene, and nuclear reprogramming factors for somatic cells that include the gene products of Oct family gene, Klf family gene, Sox family gene and Myc family gene. This publication also describes a method for producing induced pluripotent stem cells by nuclear reprogramming of somatic cells, which comprises a step of allowing the aforementioned nuclear reprogramming factors to come into contact with somatic cells

A "monocyte cell" is a mononuclear phagocyte of the peripheral blood. Monocytes vary considerably, ranging in size from 10 to 30 µm in diameter. The nucleus to cytoplasm ratio ranges from 2:1 to 1:1. The nucleus is often band shaped (horseshoe), or reniform (kindey-shaped). It may fold over on top of itself, thus showing brainlike convolutions. No nucleoli are visible. The chromatin pattern is fine, and arranged in skein-like strands. The cytoplasm is abundant and appears blue gray with many fine azurophilic granules, giving a ground glass appearance in Giemsa staining. Vacuoles may be present. More preferably, the expression of specific surface antigens is used to determine whether a cell is a monocyte cell. The main phenotypic markers of human monocyte cells include CD11b, CD11c, CD33 and CD115. Generally, human monocyte cells express CD9, CD11b, CD11c, CDw12, CD13, CD15, CDw17, CD31, CD32, CD33, CD35, CD36, CD38, CD43, CD49b, CD49e, CD49f, CD63, CD64, CD65s, CD68, CD84, CD85, CD86, CD87, CD89, CD91, CDw92, CD93, CD98, CD101, CD102, CD111, CD112, CD115, CD116, CD119, CDw121b, CDw123, CD127, CDw128, CDw131, CD147, CD155, CD156a, CD157, CD162, CD163, CD164, CD168, CD171, CD172a, CD180, CD206, CD131a1, CD213a2, CDw210, CD226, CD281, CD282, CD284, CD286 and optionally CD4, CD14, CD16, CD40, CD45RO, CD45RA, CD45RB, CD62L, CD74, CD142 and CD170, CD181, CD182, CD184, CD191, CD192, CD194, CD195, CD197, CX3CR1.

A "macrophage cell" is a cell exhibiting properties of phagocytosis. The morphology of macrophages varies among different tissues and between normal and pathologic states, and not all macrophages can be identified by morphology alone. However, most macrophages are large cells with a round or indented nucleus, a well-developed Golgi apparatus, abundant endocytotic vacuoles, lysosomes, and phagolysosomes, and a plasma membrane covered with ruffles or microvilli. The key functions of macrophages in innate and adaptive immunity are the phagocytosis and subsequent degradation of senescent or apoptotic cells, microbes and neoplastic cells, the secretion of cytokines, chemokines and other soluble mediators, and the presentation of foreign antigens (peptides) on their surface to T lymphocytes. Macrophages are derived from common myeloid progenitor cells and granulocyte-monocyte progenitor cells in the bone marrow of mammalian organisms, which ultimately develop through further progenitor stages into monocytes that then enter the peripheral bloodstream. Unlike neutrophils, with their multilobed nuclei, monocytes have kidney-shaped nuclei and assume a large cell body during further differentiation and activation. Throughout life, some monocytes adhere to and migrate through the endothelium of the capillaries into all organs, where they differentiate into resident tissue macrophages or dendritic cells (see below). Besides monocyte origin, tissue resident macrophages can also develop from early primitive macrophage progenitors of the yolk sac from before the establishment of definitive hematopoiesis, from erythroid-macrophage progenitors (EMP) of diverse hematopoietic sites of the embryo or from embryonic hematopoietic stem cell derived fetal monocytes. These embryo derived macrophages can persist into adulthood and be maintained long term independently of input from adult hematopoietic stem cells. Lymphatic tissues, such as the lymph nodes and the spleen, are particularly rich in macrophages. In some organs the macrophages carry special names, as summarized in Table 1.

### Table 1 Examples of tissue macrophages

| **Organ** | **Macrophage population** |
|---|---|
| Bone | Osteoclasts |
| Central nervous system | Microglia |
| Connective tissue | Histiocytes |
| Chorion villi | Hofbauer cells |
| Kidney | Mesangial cells |
| Liver | Kupffer cells |
| Peritoneal cavity | Peritoneal macrophages |
| Pulmonary airways | Alveolar macrophages, lung interstitium interstitial macrophages |
| Skin | Epidermal langerhans cells and dermal macrophages |
| Spleen | Marginal zone macrophages, Metallophilic macrophages, Red pulp macrophages, White pulp macrophages |

Further examples of macrophages are peritubular and interstitial testicular macrophages, cardiac macrophages from heart, adipose tissue macrophages from fat tissue, Large and small intestinal macrophages from intestine, skeletal muscle macrophages, synovial macrophages from joints, arterial adventitia macrophages, arterial intima macrophages, blood vessel associated macrophages, resident pancreatic macrophages, meningeal macrophages, pleural macrophages, omentum macrophages

In the context of the invention, the macrophage can be selected from any one of the macrophages mentioned above, preferably the macrophage is selected from the group consisting of microglia, histiocytes, Hofbauer cells, mesangial cells, Kupffer cells, peritoneal macrophages, alveolar macrophage, epidermal or dermal macrophages, marginal zone macrophages, metallophilic macrophages, Red pulp macrophages, white pulp macrophages and osteoclasts.

Macrophages are an important source of cytokines. Functionally, the numerous products can be placed into several groups: (1) cytokines that mediate a proinflammatory response, i.e. help to recruit further inflammatory cells (e.g. IL-1, II-6, TNFs, CC and CXC chemokines, such as IL-8 and monocyte-chemotactic protein 1); (2) cytokines that mediate T cell and natural killer (NK) cell activation (e.g. IL-1, IL-12, IL-15, IL-18); (3) cytokines that exert a feedback effect on the macrophage itself (e.g. IL-1, TNFs, IL-12, IL-18, M-CSF, IFNα/β, IFNγ); (4) cytokines that downregulate the macrophage and/or help to terminate the inflammation (e.g. IL-10, TGFβs), (5) cytokines important for wound healing or to support tissue stem cells (e.g. EGF, PDGF, bFGF, TGFβ) or to support blood vessel growth (e.g. VEGF) or neurons (e.g. neurotrophic factors, kinins). The production of cytokines by macrophages can be triggered by microbial products such as LPS, by interaction with type 1 T-helper cells, or by soluble factors including prostaglandins, leukotrienes and, most importantly, other cytokines (e.g. IFNγ). Generally, human macrophages express CD11c, CD11b, CD18, CD26, CD31, CD32, CD36, CD45RO, CD45RB, CD63, CD68, CD71, CD74, CD87, CD88, CD101, CD119, CD121b, CD155, CD156a, CD204, CD206 CDw210, CD281, CD282, CD284, CD286 and in a subset manner CD14, CD16, CD163, CD169 CD170, MARCO FOLR2, LYVE1. Activated macrophages further express CD23, CD25, CD69 and CD105.

A "dendritic cell" (DC) is an antigen presenting cell existing in vivo, in vitro, ex vivo, or in a host or subject, or which can be derived from a hematopoietic stem cell, a hematopoietic progenitor or a monocyte. Dendritic cells and their precursors can be isolated from a variety of lymphoid organs, e.g., spleen, lymph nodes, as well as from bone marrow and peripheral blood. The DC has a characteristic morphology with thin sheets (lamellipodia) extending in multiple directions away from the dendritic cell body. DCs express constitutively both MHC class I and class II molecules, which present peptide antigens to CD8+ and CD4+ T cells respectively. In addition, human skin and mucosal DCs also express the CD1 gene family, MHC class I-related molecules that present microbial lipid or glycolipid antigens. The DC membrane is also rich in molecules that allow adhesion of T cells (e.g. intercellular adhesion molecule 1 or CD54) or that co-stimulate T-cell activation such as B7-1 and B7-2 (also known as CD80 and CD86 respectively). Generally, DCs express CD85, CD180, CD187 CD205 CD281, CD282, CD284, CD286 and in a subset manner CD206, CD207, CD208 and CD209.

By "purified" and "isolated" it is meant, when referring to a polypeptide or a nucleotide sequence, that the indicated molecule is present in the substantial absence of other biological macromolecules. When referring to a cell or a population of cells, the term means that said cell or said population of cells is present in the substantial absence of other cells or population of cells. The term "purified" as used herein preferably means at least 75% by weight or number, more preferably at least 85% by weight or number, still preferably at least 95% by weight or number, and most preferably at least 98% by weight or number, of biological macromolecules or cells of the same type are present. An "isolated" nucleic acid molecule, which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

The terms "phagocytic cells" and "phagocytes" are used interchangeably herein to refer to a cell that is capable of phagocytosis. There are three main categories of professional phagocytes: macrophages, mononuclear cells (histiocytes and monocytes); polymorphonuclear leukocytes (neutrophils) and dendritic cells. However, there are also "non-professional" phagocytic cells known to participate in efferocytosis, efferocytosis being the process by which professional and nonprofessional phagocytes dispose of apoptotic cells in a rapid and efficient manner.

As used herein, the term "subject" denotes a human being. The phagocytic cells described herein, e.g. the monocytes, macrophages, or dendritic cells, are thus human cells.

In the context of the invention, the term "treating" or "treatment", as used herein, means reversing, alleviating, inhibiting the progress of, or preventing the disease or condition to which such term applies, or one or more symptoms of such disease or condition.

The term "non-tumorigenic" as used herein refers to cells that fail to form a visible tumor 4 weeks after intratracheal transplantation of at least 8x10⁶ cells into an immunocompromised huPAP mouse as described in example 5. If the same number of cancer cells are transplanted under the same circumstances, the cancer cells do form (a) tumor(s) during the same period that is/are visible when mice are sacrificed and the lungs opened for visual inspection.

The term "under suitable cultivation conditions" as used herein are conditions under which the phagocytic cells of the invention are able to grow. The cultivation is typically carried out in a cell culture medium supplemented with appropriate growth factors and at a suitable temperature and in a suitable controlled atmosphere. RPMI medium (RPMI derived its name from the Roswell Park Memorial Institute and is a medium frequently used for the culture of human lymphocytes) supplemented with 10% FBS (PAA-GE Healthcare, A15-101), supplemented with 100 units/ml penicillin, 100 ug/ml streptomycin (Thermo Fisher, #15140122), 2 mM GlutaMAX (Thermo Fisher, #35050038), 1 mM sodium pyruvate (Thermo Fisher, #11360-039), 50 ng/ml M-CSF (Thermo Fisher, #PHC9504), and, when indicated, 50 ng/ml GM-CSF (Peprotech, #300-03), as described in example 3, is a suitable cultivation medium. Cultivation is typically at 37°C and 5% CO2, 21% O₂.

The term "granddaughter cell" as used herein is a cell derived from a parent cell by two mitotic cell divisions.

As used herein, the process of phagocytosis refers to the uptake or engulfment of an extracellular molecule or a molecule bound to the surface of the cell. The term "being capable of phagocytosis" refers to a cell that can take up or engulf an extracellular molecule or a molecule bound to the surface of the cell. Preferred cells are professional phagocytic cells, which include, but are not limited to, neutrophils, monocytes, macrophages, dendritic cells, and/or mast cells. Non-professional phagocytes include, but are not limited to, epithelial cells, endothelial cells, fibroblasts, and/or mesenchymal cells.

The term "reactive oxygen species" as used herein relates to unstable molecules that contains oxygen and that easily react with other molecules in a cell. Typically, reactive oxygen species are free radicals. Reactive Oxygen Species are known to be a component of the killing response of immune cells to microbial invasion.

The term "activation" as used herein relates to the phenomenon that external stimuli can induce changes to cell, thereby activating it. Macrophages, for example, can be activated by cytokines such as interferon-gamma (IFN-gamma) and bacterial endotoxins, such as lipopolysaccharide (LPS). Activated macrophages undergo many changes which allow them to kill invading bacteria or infected cells. They release toxic chemicals and proteins which have toxic effects on other cells. Activated macrophages are larger, have increased metabolism, increased levels of lysosomal proteins, and a greater ability to phagocytosis and kill microbes. Activated macrophages also release proteases, neutrophil chemotatic factors; reactive oxygen species such as nitric oxide and superoxide; cytokines such as tumor necrosis factor-alpha (TNF-alpha), interleukin one and eight (IL-1 and IL-8), eicosanoids, as well as growth factors. These products of activated macrophages can result in the kind of tissue destruction which is a hallmark of inflammation

The term "adherent" as used herein relates to the property of adherent cells that they attach to a solid substrate, such as the bottom of a tissue culture flask. In contrast, suspension cells will float and grow suspended in the culture medium, so they don't need to be mechanically or chemically removed.

Lamellipodium as used herein is a projection on the leading edge of the cell. It contains a quasi-two-dimensional actin mesh. A filopodium is a finger-like structure within the lamellipodium that has spread beyond the lamellipodium frontier and thus extends from it.

The term "chromosome rearrangement" as used herein is a chromosome abnormality involving a change in the structure of a native chromosome. Usually, chromosome rearrangements are caused by a breakage in the DNA double helices at two different locations, followed by a rejoining of the broken ends to produce a new chromosomal arrangement of genes, different from the gene order of the chromosomes or the chromosomes before it or they were broken. Such changes may involve several different classes of events, like large deletions of more than 10000 base pairs, gene duplications, gene inversions and translocations, within one or between two chromosomes.

The term "karyotyping" as used herein is the analysis of the chromosomes within cells from a sample. Chromosomes are stained and the skilled person then uses a microscope to examine the size, shape, and number of chromosomes in the cells of the cell sample. Usually, the stained sample is photographed to show the arrangement of the chromosomes. Karyotypes describe the chromosome count of an organism and what these chromosomes look like under a light microscope, in particular the length of the chromosomes, the position of the centromeres within those chromosomes, the banding pattern of the stained chromosomes, any differences between the sex chromosomes, and any other physical characteristics.

The term "guide RNA" as used herein relates to "guide RNA" as used in the context of a CRISPR/Cas9 DNA editing system. The guide RNA confers target sequence specificity to the CRISPR-Cas9 system. Guide RNAs are non-coding short RNA sequences which first bind to the Cas9 enzyme and then the guide RNA sequence guides the complex via base pairing to a specific location on the DNA, where Cas9 acts as an endonuclease and cuts the target DNA strand. Examples of guide RNAs are a) a synthetic trans-activating CRISPR RNA (tracrRNA) plus a synthetic CRISPR RNA (crRNA), wherein the crRNA is designed to identify the gene target site of interest, and b) a single guide RNA (sgRNA) that combines both the crRNA and tracrRNA within a single construct.

The term "progenitor cell" as used herein relates to cells which are descendants of stem cells and which can further differentiate to create specialized cell types. There are many types of progenitor cells throughout the human body. Each progenitor cell is only capable of differentiating into cells that belong to the same tissue or organ. Some progenitor cells have one final target cell that they differentiate to, while others have the potential to terminate in more than one cell type. Progenitor cells are thus an intermediary cell type involved in the creation of mature cells in human tissues and organs, the blood, and the central nervous system. Hematopoietic progenitor cells are an intermediate cell type in blood cell development. They are immature cells that develop from hematopoietic stem cells and eventually differentiate into one of more than ten different types of mature blood cells.

The term "CD34+ multipotent progenitors" as used herein are a CD34 surface antigen expressing stem-cell enriched hematopoietic progenitor population, that are not macrophages, monocytes or dendritic cells.

The term "monoblasts" as used herein relates to committed progenitor cells in bone marrow that differentiated from myeloid progenitor cells in the process of hematopoiesis. They can mature into monocytes which, in turn, can develop into macrophages.

The term "collection of cells" as used herein relates to at least 100 cells, which cells are alive.

The term "expansion" of cells as used herein is the process of culturing cells under suitable laboratory conditions and increasing the number of living cells by mitotic divisions of the cultured cells.

The term "genetically modified" cell as used herein relates to a cell wherein the cell's DNA has been changed using biotechnological methods. For example, cells wherein the cells' DNA has been manipulated by the use of a CRISPR/Cas9 DNA editing system, wherein the manipulation has left a detectable change in the cells' DNA, are genetically modified cells.

The term "chimeric antigen receptor" as used herein is a fusion of an extracellular recognition domain (e.g. an antigen-specific targeting region), a transmembrane domain, and one or more intracellular signaling domains. Upon antigen engagement by the extracellular recognition domain, the intracellular signaling portion of the CAR can initiate an activation-related response in an immune cell, such as the release of cytolytic molecules to induce tumor cell death, etc.

The term "ex-vivo" as used herein means outside of a living body.

The term "in-vitro" as used herein means outside of a living body and within a laboratory environment. For example, cells which are cultured "in-vitro" are cultured in controlled, and often artificial, culture media.

The present invention relates to a human phagocytic cell, wherein the cell is non-tumorigenic and capable to give rise to at least four granddaughter cells under suitable cultivation conditions. By providing proliferating human phagocytic cells, and in particular proliferating professional phagocytic cells like the macrophages, monocytes or dendritic cells of the myeloid lineage, the present inventors have overcome a major limitation that has frustrated attempts to use macrophages, and in particular genetically modified macrophages, in human therapy, the limitation being how to obtain sufficiently high cell numbers for therapy from limited sources.

The human phagocytic cell of the invention may be characterized by being in a differentiated state and it expresses surface markers which are indicative of functional macrophages, such as CD45, CD64, CD11b, CD33, CD206 and CD14. A further indication for its differentiated state is that surface markers for precursor cells, such as the protein CD34, are not detectable on the surface of the phagocytic cell using flow cytometric analysis of cells stained with antibodies against the specific surface markers.

The human phagocytic cell of the invention can also be characterized by its functional features. For example, it can be characterized by being capable of phagocytosis (Liu 2020). For example, it can be characterized by being capable to produce reactive oxygen species upon activation (Thomas 2020). For example, it can be characterized by protease activity which can be detected in its lysosomes (Thomas 2020). For example, it can be characterized by being capable to take up lipids as, for example, determined by OilRedO (Rauschmeier (2019)) or BODIPY (Qiu (2016)). The human phagocytic cell of the invention can show one or more, such as all, of these functional characteristics. Aziz et al. (2009) also provide assays for functional features in the material and methods section.

The human phagocytic cell of the invention may also be characterized by negative features. For example, while it may be derived from a hematopoietic stem cell, it itself is preferably not a hematopoietic stem cell. For example, while the deletion of MAF and MAFB may alter the expression levels of responsive genes, the human phagocytic cell of the invention does preferably not comprise an additional copy of an oncogene, for example such as Hoxb8.

The human phagocytic cell of the invention may also be characterized by its morphological features. For example, it can be characterized by appearing as a large vacuolated cell in a microscope. For example, it can be characterized by appearing as a cell with adherent morphology. For example, it can it can be characterized by appearing as a cell featuring lamello- and/or filopodia. The human phagocytic cell of the invention can be characterized by one or more, such as all, of these morphological characteristics.

The human phagocytic cell of the invention is non-tumorigenic despite its capacity to proliferate. For example, it can be characterized by having 46 chromosomes only. For example, it can be characterized by the absence of any chromosome with a chromosome rearrangement. These characteristics can be, for example, determined by karyotyping using conventional cytogenetic analysis (G-banding). In tests to assess the tumorigenicity of the phagocytic cells of the invention we have observed that immunocompromised huPAP mice engrafted with our cells do not display tumours upon macroscopic inspection. Thus, the human phagocytic cell of the invention can be characterized by not forming a tumor upon transplantation into immunocompromised huPAP mice, for example as described in example 5.

A preferred human phagocytic cell of the invention is a cell, wherein both alleles of MAFB and both alleles of MAF have been rendered nonfunctional by deletions of at least 50 base pairs. The present inventors have found that inhibition of MAFB and MAF gene expression in human cells has proven surprisingly more difficult than in murine cells, in part because a genetic approach to double-deficient cells is not an option for human cells. While the use of one guide RNA per gene in CRISPR/Cas9-based approaches has in the past provided many successful examples for inactivation of gene expression of other genes, for example Chu et al. (2016), several attempts to render both MAFB and MAF genes non-functional by creating small deletions with a CRISPR/Cas9 approach and one guide RNAs for each of MAFB and MAF did not yield cells that were observed to expand in vitro. Surprisingly, the use of two guide RNAs per gene, generating larger deletions of at least 50 base pairs, was successful in providing proliferating phagocytic cells. These cells had the additional advantage of being non-tumorigenic.

The preferred human phagocytic cell of the invention can also be characterized by the nature and size of the deletions. For example, all deletions can be in exonic parts of the MAFB and MAF genes. For example, the deletions can be larger than 50 base pairs each, such as at least 100 base pairs each or at least 250 base pairs each. For example, the deletions of or within the MAFB and MAF genes can be from 100 base pairs to 10000 base pairs each, such as from 500 base pairs to 3000 base pairs each, in particular from 700 base pairs to 2000 base pairs. The human phagocytic cell of the invention can show one or more, such as all, of these deletions.

The preferred human phagocytic cell of the invention can also be characterized by the location of the deletion in a particular chromosome. For example, the MAFB deletions are preferred within the region on human chromosome 22 from 40685000 to 40690000, such as from 40685200 to 40689800, for example from 40685400 to 40689600, such as from 40685600 to 40689400 and in particular from 40685700 to 40689300. For example, the MAF deletions are preferred within the region on human chromosome 16 from 79593000 to 79602000, such as from 79593200 to 79601500, for example from 79593400 to 79601100, and in particular from 79593600 to 79600900.

The human phagocytic cell of the invention may be prepared by manipulation of a cell. Cells, from which the phagocytic cell of the invention can be derived, can be cells which are progenitors for the development of human macrophages, for example cells selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow; mobilized monoblasts, myeloid or CD34+ multipotent progenitors from adult blood; and monocytes, monoblasts, myeloid or CD34+ progenitors from extramedullary hematopoiesis. The human phagocytic cell comprised in the collection of human phagocytic cells of the invention can also be derived from macrophages, in particular macrophages which are relatively easily accessible such as alveolar macrophages or macrophages from fat tissue.

The preparation of human induced pluripotent stem cells is by now well established in the art. Isogai et al. (2018), for example, describe the preparation of iPS cells from human blood monocytes. Fusaki N., et al. (2009) describe the neneration of human iPS cell lines with a modified Sendai virus.

Human mononuclear phagocytes for the deletion of MafB and cMaf genes can also be derived from primary human blood monocytes, which can be obtained by leukapheresis and elutriation, or from monocyte derived macrophages, for which culture conditions are well known to the art. Human macrophages can also be obtained from bronchoalveolar lavage and by differentiation of CD34+ hematopoietic stem and progenitor cells obtained from umbilical cord blood, stem cell mobilized peripheral blood or bone marrow, using differentiation protocols well known to the art.

Cas9 and gRNAs can be expressed by publicly and commercially available DNA expression plasmids well known in the art (for example Santa Cruz Sc-418922, https://www.scbt.com/de/p/control-crispr-cas9-plasmid). DNA expression plasmids can be introduced into human mononuclear phagocyte target cells by electroporation or lipid-based transfection protocols well known to the art. Cas9 and gRNA can also be introduced into target cells as a ribonucleic/protein complex by electroporation. Cas9/gRNA mediated gene editing has been demonstrated in mononuclear phagocytes using such methods (Zhag et al. (2020); Wang et al. (2018)) and in human CD34+ hematopoietic stem and progenitor cells differentiating to macrophages (Scharenberg et al. (2020)). MafB and cMaf genes could also be deleted utilizing engineered site directed recombinases (Lansing et al. (2020); Karpinsky et al. (2016)), which can be introduced by methods known to the art, such as electroporation of the protein, coding mRNA or DNA expression plasmids, and which have been used for gene editing in mononuclear phagocytes (Shi et al. (2018)).

Gonzalez F. et al. (2014) describe an iCRISPR platform for rapid, multiplexable and inducible genome editing in human pluripotent stem cells based on the introduction of a doxycycline-inducible Cas9 expression cassette into the AAVS1 locus. This strategy can be used to introduce an inducible Cas9-expression cassette into a wide variety of self-generated or publicly available human iPS cell lines. Such modified cell lines can then be used for targeting MAF and MAFB.

The present invention therefore also relates to a method for generating to a proliferating human phagocytic cell, wherein the cell is non-tumorigenic, the method comprising the step of
effecting two double strand breaks each a1) within the region on human chromosome 22 from 40685000 to 40690000 and a2) within the region on human chromosome 16 from 79593000 to 79602000, in a human phagocytic cell; and b) selecting cells with deletions in both alleles of MAFB and in both alleles of MAF.

The method preferably also comprises the step c) of selecting for proliferating cells.

Preferred target locations for the double strand breaks are within the region on human chromosome 22 from from 40685700 to 40689300 and within the region on human chromosome 16 from 79593600 to 79600900.

As described above, the double strand breaks are typically effected by site-specific endonucleases or recombinases, for example Cas-9 in combination with at least four guide RNAs, wherein at least two of the at least four guide RNAs are directed at the MAFB locus on chromosome 22 and at least two of the at least four guide RNAs are directed at the MAF locus on chromosome 16.

Preferably the human phagocytic cell used as the target cell for step a) is an iPS cell, a monocyte or a macrophage, in particular wherein the human phagocytic cell is selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow, mobilized monoblasts, myeloid or CD34+ multipotent progenitors from adult blood; monocytes, monoblasts, myeloid or CD34+ progenitors from extramedullary hematopoiesis; alveolar macrophages; and adipose tissue macrophages.. One example for a target cell for step a) is an induced pluripotent stem cell.

After cells with deletions in both alleles of MAFB and in both alleles of MAF have been selected, they are typically cultured in a suitable medium, for example in the presence of M-CSF. An example for cultivation conditions is described in example 3.

Some aspects of the invention are best described when looking at cells in the context of a collection of cells, for example a cell culture. In an alternative embodiment, the present invention therefore relates to a collection of human phagocytic cells, wherein the cells are non-tumorigenic, and wherein the number of cells increases at least 4-fold under suitable cultivation conditions. Such a collection of cells will be referred to below as "the collection of human phagocytic cells of the invention". The collection of human phagocytic cells of the invention is preferably a collection of many cells, such as where the number of cells is at least 1000, at least 10000 or at least 100000.

The collection of human phagocytic cells of the invention, under suitable cultivation conditions, can be described by being capable of an increase in cell number of at least 4-fold, for example of at least 10-fold, at least 15-fold or even at least 20-fold. The collection of human phagocytic cells of the invention, under suitable cultivation conditions, can be described by being capable of an increase in cell number of preferably from 4-fold to 100-fold, for example from 10-fold to 80-fold, such as from 15-fold to 60-fold. This increase in cell number corresponds to the increases in cell number observed in the final differentiation phase.

The increase in cell number is even higher when compared to the number of cells used as the input for the initial embryonic body formation, namely an increase in cell number over input iPS cells of from 20-fold to 10000-fold, such as from 50-fold to 5000-fold, for example from 100-fold to 3000-fold.

The collection of human phagocytic cells of the invention can also be characterized by the type of cells comprised in the collection. The present invention therefore also relates to the collection of human phagocytic cells of the invention, wherein the phagocytic cell is a professional phagocytic cell such as a myeloid cell, and in particular a macrophage, monocyte or dendritic cell.

The collection of human phagocytic cells of the invention can be characterized by comprising human phagocytic cells which are in a differentiated state and/or which express surface markers which are indicative of functional macrophages, such as CD45, CD64, CD11b, CD33, CD206 and CD14. A further indication for the differentiated state of cells comprised in the collection of human phagocytic cells of the invention is that surface markers for precursor cells, such as the protein CD34, are not detectable on the surface of the human phagocytic cells comprised in the collection.

The collection of human phagocytic cells of the invention can be characterized by comprising human phagocytic cells which can be characterized by their functional features. For example, the human phagocytic cells comprised in the collection can be characterized by being capable of phagocytosis. For example, the human phagocytic cells comprised in the collection can be characterized by being capable to produce reactive oxygen species upon activation. For example, the human phagocytic cells comprised in the collection can be characterized by protease activity which can be detected in its lysosomes. For example, the human phagocytic cells comprised in the collection can be characterized by being capable to take up lipids. The human phagocytic cell of the invention can show one or more, such as all, of these functional characteristics.

The human phagocytic cells comprised in the collection of cells of the invention can show one or more, such as all, of these functional characteristics.

The collection of human phagocytic cells of the invention may be characterized by comprising human phagocytic cells which can be characterized morphologically. For example, the human phagocytic cells comprised in the collection can be characterized by appearing as a large vacuolated cell in a microscope. For example, the human phagocytic cells comprised in the collection can be characterized by appearing as a cell with adherent morphology. For example, the human phagocytic cells comprised in the collection can be characterized by appearing as a cell featuring lamello- and/or filopodia. The human phagocytic cells comprised in the collection can be characterized by one or more, such as all, of these morphological characteristics.

The human phagocytic cells comprised in the collection of human phagocytic cells of the invention are non-tumorigenic despite their capacity to proliferate. For example, the collection of human phagocytic cells of the invention can be characterized by consisting of cells having 46 chromosomes only. For example, the collection can be characterized by the absence of cells having a chromosome with a chromosome rearrangement. These characteristics can be, for example, determined by karyotyping of a sufficient number of cells comprised in the collection, such as at least 30 cells, at least 40 cells or at least 50 cells. In tests to assess the tumorigenicity of the human phagocytic cells comprised in the collection of human phagocytic cells of the invention we have observed that intratracheal instillation of at least 8x10⁶ cells into the lung of immunocompromised huPAP-mice did not lead to the formation of visible tumors in the lungs of the animals. Thus, the collection of human phagocytic cells of the invention can be characterized by not forming a tumor upon transplantation into the lungs of immunocompromised of huPAP mice when the lungs of the animals are inspected after four weeks.

A preferred human phagocytic cell comprised in the collection of human phagocytic cells of the invention is a cell, wherein both alleles of MAFB and both alleles of MAF have been rendered nonfunctional by deletions of at least 50 base pairs.

The preferred human phagocytic cell comprised in the collection of human phagocytic cells of the invention can also be characterized by the nature and size of the deletions. For example, all deletions can be in exonic parts of the MAFB and MAF genes. For example, the deletions can be larger than 50 base pairs each, such as at least 100 base pairs each or at least 250 base pairs each. For example, the deletions within the MAFB and MAF genes can be from 100 base pairs to 10000 base pairs each, such as from 500 base pairs to 3000 base pairs each. The human phagocytic cell comprised in the collection of human phagocytic cells of the invention can show one or more, such as all, of these deletions.

The preferred human phagocytic cell comprised in the collection of human phagocytic cells of the invention can also be characterized by the location of the deletion in a particular chromosome. For example, the MAFB deletions are preferred within the region on human chromosome 22 from 40685000 to 40690000, such as from 40685200 to 40689800, for example from 40685400 to 40689600, such as from 40685600 to 40689400 and in particular from 40685700 to 40689300. For example, the MAF deletions are preferred within the region on human chromosome 16 from 79593000 to 79602000, such as from 79593200 to 79601500, for example from 79593400 to 79601100, and in particular from 79593600 to 79600900.

Cells, from which the human phagocytic cell comprised in the collection of human phagocytic cells of the invention can be derived, can be cells which are progenitors for the development of human macrophages, for example cells selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow; mobilized monoblasts, myeloid or CD34+ multipotent progenitors from adult blood; and monocytes, monoblasts, myeloid or CD34+ progenitors from extramedullary hematopoiesis. The human phagocytic cell comprised in the collection of human phagocytic cells of the invention can also be derived from macrophages, in particular macrophages which are relatively easily accessible such as alveolar macrophages or macrophages from fat tissue.

The human phagocytic cell of the invention and/or the collection of human phagocytic cells of the invention can be used as such in medicine, for example for the treatment of diseases like a cancer, an immune-deficiency, a chronic or an acute injury, such as central nervous system injury - for example spinal cord injury-acute injury such as ischemic stroke or myocardial infarction, chronic injury such as hepatic fibrosis, a wound, such as a chronic wound, a degenerative disease, an autoimmune disease, such as type 1 diabetes, rheumatoid arthritis or osteoarthritis, a chronic inflammatory disease, atherosclerosis, poly- and osteo-arthritis, osteoporosis, an infectious disease (e.g. infections by virus, or bacteria), and a metabolic disease.

The collection of human phagocytic cells of the invention may also be expanded under suitable conditions until a desired cell number is achieved in the collection. After an exhaustive expansion phase, the resulting collection of human phagocytic cells obtainable by such an expansion can also be used in medicine, for example for the treatment of the diseases mentioned above, regardless of whether this resulting collection of cells is still capable of further proliferation or not.

For certain medical uses the human phagocytic cell of the invention and/or the human phagocytic cell comprised by the collection of human phagocytic cells of the invention may be further modified.

The invention therefore also relates to a human phagocytic cell of the invention, wherein the phagocytic cell is genetically modified at a further locus other than MAFB or MAF. The invention also relates to a collection of human phagocytic cells of the invention, wherein the human phagocytic cells comprised by the collection are genetically modified at a further locus other than MAFB or MAF. The invention also relates to a collection of human phagocytic cells obtainable by expansion of a collection of human phagocytic cells of the invention, wherein the human phagocytic cells comprised by the collection are genetically modified at a further locus other than MAFB or MAF.

In particular if allogenic macrophages are to be used for cell therapy, it will be important to have the allogenic macrophages genetically modified such that they elicit a lower immune response in the patient to which they are intended to be administered. By transferring immune cells in an allogeneic setting, there is the risk of rejection of the therapeutic cells via the recognition of mismatched HLA (major and minor), by the recipient's immune system.

The invention therefore relates to a human phagocytic cell of the invention as described above, and/or the collection of human phagocytic cells of the invention, wherein the phagocytic cell(s) lack functional expression of the Major Histocompatibility Complex (MHC) genes. WO2019/135879 A1 describes macrophage precursors that are deficient in the expression of a functional MHC and methods for how to obtain them. WO2019/135879 A1 is herein incorporated by reference, and the present invention specifically relates also to cells of claims 1 to 26 of WO2019/135879 A1 wherein every reference to a macrophage or macrophage precursor cell in the claims of WO2019/135879 A1 is to be replaced by "a human phagocytic cell of the present invention" or "a human phagocytic cell comprised by the collection of human phagocytic cells of the present invention", and wherein every reference to "precursor cells" or "immortal cell lines" is ignored. In the context of WO2019/135879 A1 providing disclosure for the present invention, claim 1 of WO2019/135879 A1 would be understood to disclose "A cell lacking functional expression of MHC genes, wherein the cell is the human phagocytic cell of the present invention, i.e. a human phagocytic cell, wherein the cell is non-tumorigenic and capable to give rise to at least four granddaughter cells under suitable cultivation conditions, and all variations herein where the human phagocytic cell of the present invention is more specifically described.

For example, the human phagocytic cell(s) of the invention can be deficient for one or several HLA-I genes (human lymphocyte antigen I), for example HLA-A, HLA-B and/or HLA-C. Also, allogenic cells that are B2M deficient elicit a lower immune response. If B2M is deficient, it is preferred that the phagocytic cell expresses HLA-E, HLA-F and HLA-G.

The present invention also relates to the human phagocytic cell of the invention, wherein the phagocytic cell comprises a chimeric antigen receptor. The invention also relates to a collection of human phagocytic cells of the invention, wherein the human phagocytic cells comprised by the collection comprise a chimeric antigen receptor. The invention also relates to a collection of human phagocytic cells obtainable by expansion of a collection of human phagocytic cells of the invention, wherein the human phagocytic cells comprised by the collection comprise a chimeric antigen receptor.

In autologous settings, i.e. in situations where cells derived from the subject to be treated him- or herself, the functional inactivation of the MHC is not necessary. Therefore, chimeric receptors can be introduced into the phagocytic cell(s) of the present invention and/or into the human phagocytic cells comprised by the collection of human phagocytic cells of the invention directly. Again, WO2019/135879 A1 describes macrophages or macrophages comprising chimeric receptors in detail, for example in claims 14 to 26. The present invention specifically relates also to cells of claims 14 to 26 of WO2019/135879 A1 wherein every reference to a macrophage or macrophage precursor cell in the claims of WO2019/135879 A1 is to be replaced by "a human phagocytic cell of the present invention" or "a human phagocytic cell comprised by the collection of human phagocytic cells of the present invention", and wherein every reference to "precursor cells" or "immortal cell lines" is ignored and wherein every back-reference to claim 1 is ignored. In the context of WO2019/135879 A1 providing disclosure for the present invention, claim 14 of WO2019/135879 A1 would be understood to disclose "A cell, wherein the cell comprises a chimeric receptor, the chimeric receptor comprising: a cytoplasmic domain; a transmembrane domain; and an extracellular domain; wherein the cytoplasmic domain comprises a cytoplasmic portion of a receptor that when activated polarizes a macrophage; wherein a wild-type protein comprising the cytoplasmic portion does not comprise the extracellular domain, wherein the cell is the human phagocytic cell of the present invention, i.e. a human phagocytic cell, wherein the cell is non-tumorigenic and capable to give rise to at least four granddaughter cells under suitable cultivation conditions, including all variations herein where the human phagocytic cell of the present invention is more specifically described.

For example, the cytoplasmic portion of the chimeric receptor may comprise a cytoplasmic domain from a toll-like receptor, myeloid differentiation primary response protein (MYD88) (SEQ ID NO: 19 in WO2019/135879 A), toll-like receptor 3 (TLR3) (SEQ ID NO: I in WO2019/135879 A), toll-like receptor 4 (TLR4) (SEQ ID NO:3 in WO2019/135879 A), toll-like receptor 7 (TLR7) (SEQ ID NO:7 in WO2019/135879 A), toll-like receptor 8 (TLR8) (SEQ ID NO:9 in WO2019/135879 A), toll-like receptor 9 (TLR9) (SEQ ID NO: 11 in WO2019/135879 A), myelin and lymphocyte protein (MAL) (SEQ ID NO:2I in WO2019/135879 A), interleukin-I receptor- associated kinase 1 (IRAK1) (SEQ ID NO:23 in WO2019/135879 A), low affinity immunoglobulin gamma Fc region receptor III-A (FCGR3A) (SEQ ID NO: 15 in WO2019/135879 A), low affinity immunoglobulin gamma Fc region receptor II-a (FCGR2A) (SEQ ID NO: 13 in WO2019/135879 A), high affinity immunoglobulin epsilon receptor subunit gamma (FCER1G) (SEQ ID NO: 19 in WO2019/135879 A), or sequences having at least 90% sequence identity to a cytoplasmic domain of any one of the foregoing.

It has been difficult to provide compositions comprising a large number of human non-tumorigenic phagocytic cells, such as macrophages, monocytes or dendritic cells, in the past. This is because the human phagocytic cells which have previously been isolated, such as macrophages, monocytes or dendritic cells, had no significant potential, if at all, to proliferate ex-vivo. This meant, for example, that therapeutic approaches which rely on isogenic macrophages, were limited to the numbers of precursor cells previously isolated from the patient.

In Nature Biotechnology, Vol.38, May 2020: 509-511, E. Dolgin discusses the advantages and limitations of therapies which are based on CAR-macrophages. While CAR-expressing human macrophages can be generated by very efficient transduction with viral vectors, unlike T-cells (which are easily expanded and on which successful CAR-T anticancer-treatments are based) monocyte-derived human macrophages do not proliferate ex vivo, describing the dilemma as "So, what you put in is basically what you get out.". "That presents a hurdle in terms of the number of cells you have to start with." However, the number of CAR-modified macrophages needed for efficient CAR-M therapy is at least around 10⁸ to 10⁹ per patient and thus the provision of human phagocytic cells which are non-tumorigenic, but capable to proliferate, would make it significantly easier to obtain the cell numbers needed for therapies based on such cells, such as therapies based on CAR-macrophages.

The invention therefore also relates to a composition comprising a human phagocytic cell of the invention as described above. The human phagocytic cells of the invention enable the preparation of compositions comprising, for example, from 10⁷ to 10¹² human phagocytic cells of the invention, such as from 10⁸ to 10¹¹, for example from 5x10⁸ to 5x10¹⁰. The ability to generate compositions with such high cell numbers allows the preparation of a pharmaceutical composition comprising human phagocytic cells of the invention.

The invention therefore also relates to a pharmaceutical composition comprising human phagocytic cells of the invention, such as a pharmaceutical composition comprising from 10⁷ to 10¹² human phagocytic cells of the invention, such as from 10⁸ to 10¹¹, for example from 5x10⁸ to 5x10¹⁰ human phagocytic cells of the invention. The invention also relates to a pharmaceutical composition comprising a collection of human phagocytic cells of the invention. The invention also relates to a pharmaceutical composition comprising a collection of human phagocytic cells obtainable by expansion of a collection of human phagocytic cells of the invention, such as a pharmaceutical composition comprising from 10⁷ to 10¹² human phagocytic cells, such as from 10⁸ to 10¹¹, for example from 5x10⁸ to 5x10¹⁰ human phagocytic cells.

The exact cell number in the pharmaceutical compositions will depend in the intended therapy. Some therapeutic protocols, for example based on CAR-macrophophages, have used from 10⁶ to 10⁸ CAR-macrophages, but the ability to generate compositions with higher cell numbers will enable therapeutic protocols using higher numbers of CAR-macrophages, such as from 10⁷ to 10⁹ CAR-macrophages or from 10⁸ to 10¹⁰ CAR-macrophages or from 10⁸ to 10¹¹.

Pharmaceutical compositions of the present invention comprise a therapeutically effective number of human phagocytic cells, and a pharmaceutically acceptable carrier or excipient. By a "therapeutically effective amount" of a cell as above described is meant a sufficient amount of said cell to treat a disease or disorder at a reasonable benefit/risk ratio applicable to any medical treatment. It will be understood, however, that the total daily usage of compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific cells employed; and like factors well known in the medical arts.

Pharmaceutical compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Pharmaceutical compositions of the present invention are preferably formulated for intratracheal, intraperitoneal or intravenous administration. The cell compositions described herein may also be administered multiple times at these dosages. Infusion techniques in immunotherapy are commonly known (e.g., Rosenberg et al., New Eng. J. of Med. 319: 1676, 1988). A physician can readily determine the optimal dosage and treatment regime for any particular patient by monitoring the patient for signs of disease and adjusting the treatment accordingly.

According to the present invention, human phagocytic cells, such as human monocytes, human macrophages and human dendritic cells, can be effectively generated ex-vivo and the ability to obtain a large number of ex-vivo produced monocytes, macrophages and dendritic cells opens new opportunities for their use in human therapy. The invention therefore also relates to the use of a human phagocytic cell of the invention as described above and/or a pharmaceutical composition, as described above, comprising human phagocytic cells of the invention, as a medicament. The invention also relates to the use of a collection of human phagocytic cells of the invention as described above and/or a pharmaceutical composition, as described above, comprising a collection of human phagocytic cells of the invention, as a medicament. The invention also relates to the use of a collection of human phagocytic cells obtainable by expansion of a collection of human phagocytic cells of the invention, as a medicament.

The mononuclear phagocyte system (monocyte, macrophages and dendritic cells) represents a population of immune cells that circulate in the blood as monocytes, perform antigen presenting functions as dendritic cells in the periphery and lymphoid organs and populate tissues as macrophages. In particular macrophages are present in all organs and beyond pathogen control assume homeostatic functions within the host. Said system is involved in essentially every disease process in which there is persistent tissue injury or metabolic disturbance. Macrophages and monocytes mediate acute as well as chronic inflammation, and promote repair through removal of dead cells and fibrin by phagocytosis and fibrinolysis, induce blood vessel ingrowth (angiogenesis) and modulate fibroblast invasion and production of extracellular matrix. They also maintain tissue integrity, architecture and function by phagocytosing cellular and extracellular material, for example axon pruning by microglia, surfactant clearing by alveolar macrophages and resrorption of mineral material in bones by osteoclast. They also serve important functions in repair and regeneration through trophic and modulatory functions on different tissue specific adult stem and progenitor cells by paracrine action or direct interaction in the respective niche. They produce mediators that mobilize systemic responses of the host including fever, release and catabolize stress and other hormones, increase metabolic activity of other cells, and influence blood flow to tissues and capillary permeability. The macrophages themselves display considerable heterogeneity in their functions, often expressing activators as well as inhibitors of a property, e.g. proteolytic activity, or pro- and anti-inflammatory cytokine production, depending on the evolution of a particular host response. The human phagocytic cells of the invention are therefore useful in a method for treating a human subject affected with a disease that can benefit from administration of professional human phagocytic cells. Diseases that benefit from administration of professional human phagocytic cells are, for example, a disease resulting from a deficiency in professional human phagocytic cells, or a disease where a specific beneficial phagocyte function is underdeveloped, inhibited or inactivated, or a disease where a specific undesirable phagocyte function is overdeveloped, increased or activated. Without wishing to be bound by any theory, mechanistically this may be by competitive replacement of a pathologic phagocyte population or by compensatory beneficial function of the transplanted phagocyte population.

The method of treatment comprises administering a human phagocytic cell of the invention as described above and/or a pharmaceutical composition, as described above, comprising human phagocytic cells of the invention, to said subject. Likewise, the collection of human phagocytic cells of the invention is useful in a method for treating a human subject affected with a disease that can benefit from administration of professional human phagocytic cells, which method comprises administering a collection of human phagocytic cells of the invention as described above and/or a pharmaceutical composition, as described above, comprising a collection of human phagocytic cells of the invention, to said subject. Likewise, the collection of human phagocytic cells obtainable by expansion of a collection of human phagocytic cells of the invention is useful in a method for treating a human subject affected with a disease that can benefit from administration of professional human phagocytic cells, which method comprises administering a collection of human phagocytic cells obtainable by expansion of a collection of human phagocytic cells of the invention as described above and/or a pharmaceutical composition, as described above, comprising a collection of human phagocytic cells obtainable by expansion of a collection of human phagocytic cells of the invention, to said subject.

The number of cells in those treatments should be selected such that it is sufficient for the intended treatment.

A further aspect of the invention is a human phagocytic cell as described above or a pharmaceutical composition comprising human phagocytic cells of the invention as described above for use in the treatment of a disease selected from the group consisting of a cancer, an immune-deficiency, a chronic or an acute injury, such as central nervous system injury - for example spinal cord injury - acute injury such as ischemic stroke, hepatic injury or myocardial infarction, a wound, such as a chronic wound, a degenerative disease, an autoimmune disease, such as type 1 diabetes, Crohn's disease, rheumatoid arthritis or osteoarthritis, an acute inflammatory disease- for example acute respiratory distress syndrome (ARDS)-, a chronic inflammatory disease- for example chronic obstructive pulmonary disease (COPD), fibrotic disease- for example lung fibrosis or liver cirrhosis-, atherosclerosis, poly- and osteo-arthritis, osteoporosis, an infectious disease (e.g. infections by virus, or bacteria), and a metabolic disease. The invention also relates to the collection of human phagocytic cells of the invention and/or the collection of human phagocytic cells obtainable by expansion of a collection of human phagocytic cells of the invention for use in these treatments.

Immunodeficiencies include acquired or genetic in origin, or as a result of radiotherapy/chemotherapy. Also, the invention offers the possibility for the development of antigen-specific cancer immunotherapies.

A human phagocytic cell as described above or a pharmaceutical composition comprising human phagocytic cells of the invention as described above, and in particular wherein the human phagocytic cell of the invention is a macrophage, may be useful for the treatment of patients experiencing myeloid cytopenia. For example, patients on chemotherapy with anticancer agents such as cyclophosphamide (CP) experience a strong reduction in the size of tissue macrophage populations that accompanies blood leukopenia. These patients are thus especially susceptible to opportunistic infections, including gram-negative bacterial pneumonia. Such opportunistic infections are a common cause of death in cancer patients who are undergoing chemotherapy (Santosuosso M, et al. 2002). Therefore, a human phagocytic cell as described above or a pharmaceutical composition comprising human phagocytic cells of the invention as described above, and in particular wherein the human phagocytic cell of the invention is a macrophage, may be useful for the treatment of subjects who have undergone a chemotherapy. The skilled person will appreciate that the collection of human phagocytic cells of the invention and/or the collection of human phagocytic cells obtainable by expansion of a collection of human phagocytic cells of the invention may also be useful for this treatment.

Macrophages may inhibit precursor cells apoptosis in a cell to cell contact and may serve as stromal support for efficient cellular engraftment for tissue repair (see for example document WO2005014016). In particular macrophages could inhibit myogenic precursor cells apoptosis. Specific macrophages may also provide a stem cell activating niche, for example during muscle injury, by directly providing cues for the stem cells to engage in the repair process (Ratnayake D. et al. (2021), Nature 592(7849):281-287). Therefore, a human phagocytic cell as described above or a pharmaceutical composition comprising human phagocytic cells of the invention as described above, and in particular wherein the human phagocytic cell of the invention is a macrophage, may be useful for the treatment of lesions such as bone or muscular lesion, possibly resulting from a disease or an injury. It can be for example a bone fracture, or a torn muscle. Said lesion may also be a cardiac lesion or injury. In particular, it can be for example myocardial infarction, heart insufficiency, coronary thrombosis, dilated cardiomyopathy or any cardiomyocyte dysfunction subsequent to, or resulting from, any genetic defect. Therefore, a human phagocytic cell as described above or a pharmaceutical composition comprising human phagocytic cells of the invention as described above, and in particular wherein the human phagocytic cell of the invention is a macrophage, may be useful for the treatment of acute cardiac insufficiencies with bad prognostic despite progress in treatments, such as infiltrative cardiomyopathy, or cardiomyopathy due to anthracyclin toxicity or cardiomyopathy secondary to HIV infection. The skilled person will appreciate that the collection of human phagocytic cells of the invention and/or the collection of human phagocytic cells obtainable by expansion of a collection of human phagocytic cells of the invention may also be useful for this treatment.

A human phagocytic cell as described above or a pharmaceutical composition comprising human phagocytic cells of the invention as described above, and in particular wherein the human phagocytic cell of the invention is a macrophage, may be also useful for the treatment of spinal cord injury. A therapy for complete spinal cord injury (SCI) may indeed comprise the administration of autologous grafts of macrophages that have been educated to a wound-healing phenotype by co-incubation with skin tissue (Schwartz M et al. 2006). The skilled person will appreciate that the collection of human phagocytic cells of the invention and/or the collection of human phagocytic cells obtainable by expansion of a collection of human phagocytic cells of the invention may also be useful for this treatment.

Macrophages are essential for wound healing and thus a human phagocytic cell as described above or a pharmaceutical composition comprising human phagocytic cells of the invention as described above, and in particular wherein the human phagocytic cell of the invention is a macrophage, may be useful for enhancing wound healing and/or repairing tissue damage. The skilled person will appreciate that the collection of human phagocytic cells of the invention and/or the collection of human phagocytic cells obtainable by expansion of a collection of human phagocytic cells of the invention may also be useful for this treatment. The wound healing process has indeed 3 phases. During the inflammatory phase, numerous enzymes and cytokines are secreted by the macrophage. These include collagenases, which clear the wound of debris, interleukins and tumor necrosis factor (TNF), which stimulate fibroblasts (to produce collagen) and promote angiogenesis; and transforming growth factor α (TGFα), which stimulates keratinocytes. This step marks the transition into the process of tissue reconstruction, ie, the proliferative phase.

Macrophages play a major role in chronic inflammatory and auto-immune disease and usually are activated or hyperactivated in a specific way under these conditions. Human phagocytic cells of the invention may be amplified and the resulting human phagocytic cells, which at that stage may or may not remain proliferative, may be genetically modified, pharmacologically treated or alternatively activated or stimulated by bio-active molecules such as cytokines or growth factors to present a desirable macrophage phenotype before introduction into a subject to compete with and supplant macrophages with undesired activity. The skilled person will appreciate that the collection of human phagocytic cells of the invention and/or the collection of human phagocytic cells obtainable by expansion of a collection of human phagocytic cells of the invention may also be useful for this treatment.

Cancer is the second leading cause of death in most developed countries accounting for about 150,000 and 550,000 deaths each year in France and USA, respectively. Despite the fact that more than half of all cancer cases can be cured, the average cancer patient, not curable by surgery or radiotherapy, still has only a low chance of being cured by any other treatment. It has long been hypothesized and it is now recognized that the immune system plays a role in cancer surveillance and in the prevention of tumors.

For example, it has been shown that activated macrophages phagocyte and kill tumor cells in vitro and can have antitumor activity in vivo. Moreover, dendritic cells (DC) prime naive T cells to become effector cells able to provide long-term protection against tumor recurrence. Cell therapy based on these two types of cells appears therefore as a promising tool to treat cancer patients. Therefore, a human phagocytic cell as described above or a pharmaceutical composition comprising human phagocytic cells of the invention as described above, and in particular wherein the human phagocytic cell of the invention is a macrophage or a dendritic cell, may be useful for treating cancer diseases. The skilled person will appreciate that the collection of human phagocytic cells of the invention and/or the collection of human phagocytic cells obtainable by expansion of a collection of human phagocytic cells of the invention may also be useful for this treatment.

E. Dolgin (2020) discusses the therapeutic utility of CAR-expressing phagocytic cells, an in particular CAR-expressing macrophages, for the treatment of solid tumors based on positive results obtained from cancer mouse models. Thus, the present invention also relates to the collection of human phagocytic cells of the invention and/or the collection of human phagocytic cells obtainable by expansion of a collection of human phagocytic cells of the invention, wherein the phagocytic cell comprises a chimeric antigen receptor, for the treatment of cancer, and in particular for the treatment of solid tumors.

Dendritic cells may be induced to mature (maturing DC) through a short treatment with a bacterial extract and interferon-gamma. Furthermore, therapeutic vaccination against tumors has been shown to provide long-term protection in animal models. Tumor (or tumor cell line) lysates constitute an attractive source of multiple tumor antigens to trigger both CD8 and CD4 T cell responses. Pulsing dendritic cells with such tumor antigens represents a tool for the treatment of cancer.

Therefore, a human phagocytic cell as described above or a pharmaceutical composition comprising human phagocytic cells of the invention as described above, and in particular wherein the human phagocytic cell of the invention is a dendritic cell, may be useful for preparing pharmaceutical compositions comprising macrophages and/or dendritic cells for treating cancer. The skilled person will appreciate that the collection of human phagocytic cells of the invention and/or the collection of human phagocytic cells obtainable by expansion of a collection of human phagocytic cells of the invention may also be useful for this treatment.

In treating cancer, for example, dendritic cells can be pulsed with tumor antigens and administered to a patient to treat, for example, established tumors, or to prevent tumor formation, as discussed above.

In another embodiment a dendritic cell, wherein the dendritic cell is derived from a human phagocytic cell of the invention, may be fused to a cancer cell and therefore can be administered to the patient, wherein the fused dendritic cell will, in its role as an antigen-presenting cell, present the antigen to the immune system. Dendritic cells can be fused with other cells, e. g., cancer cells, by any method known in the art. For example, methods for fusing dendritic cells with cancer cells, as well as methods for administering them to animals have been described in Gong et al. (Nat. Med. 3 (5): 558-561 (1997)) and Guo et al. (Science 263: 518-520 (1994) ). The skilled person will appreciate that the collection of human phagocytic cells of the invention and/or the collection of human phagocytic cells obtainable by expansion of a collection of human phagocytic cells of the invention may also be useful for this treatment.

Similar vaccination protocols may be applied to infectious diseases by loading the dendritic cells with pathogen (viral, bacterial or parasite) antigen.

### The invention is further exemplified by the following embodiments

1. A human phagocytic cell, wherein the cell is non-tumorigenic and capable to give rise to at least four granddaughter cells ex vivo under suitable cultivation conditions.
2. The human phagocytic cell of embodiment 1, wherein the phagocytic cell is a professional phagocytic cell.
3. The human phagocytic cell according to any one of embodiments 1 to 2, wherein the phagocytic cell is a myeloid cell.
4. The human phagocytic cell according to embodiment 3, wherein the phagocytic cell is a macrophage, monocyte or dendritic cell.
5. The human phagocytic cell according to any one of embodiments 1 to 4, wherein the phagocytic cell is characterized by expression of a surface protein selected from the group consisting of CD45, CD64, CD11b, CD33, CD206 and CD14.
6. The human phagocytic cell according to any one of embodiments 1 to 5, wherein the phagocytic cell is characterized in that the protein CD34 is not detectable on the surface of the phagocytic cell.
7. The human phagocytic cell according to any one of embodiments 1 to 6, wherein the phagocytic cell is characterized by its capacity to perform phagocytosis, by its capacity to produce reactive oxygen species upon activation, by protease activity in its lysosomes, and/or by its capacity to take up lipids.
8. The human phagocytic cell according to any one of embodiments 1 to 7, wherein the phagocytic cell is a large vacuolated cell and/or a cell with adherent morphology and/or a cell featuring lamello- and/or filopodia.
9. The human phagocytic cell according to any one of embodiments 1 to 8, wherein the phagocytic cell has 46 chromosomes.
10. The human phagocytic cell according to embodiment 9, wherein the phagocytic cell does not contain a chromosome with a chromosome rearrangement.
11. The human phagocytic cell according to any one of embodiments 1 to 10, wherein the phagocytic cell is a differentiated cell.
12. The human phagocytic cell according to any one of embodiments 1 to 11, wherein the phagocytic cell does not form a tumor upon transplantation into the lung of huPAP mice.
13. The human phagocytic cell according to any one of embodiments 1 to 11, wherein both alleles of MAFB and both alleles of MAF have been rendered nonfunctional by deletions of at least 50 base pairs.
14. The human phagocytic cell according to embodiment 13, wherein all deletions comprise exonic DNA.
15. The human phagocytic cell according to any one of embodiments 13 to 14, wherein the deletions are at least 100 base pairs each.
16. The human phagocytic cell according to any one of embodiments 13 to 15, wherein the deletions are at least 250 base pairs each.
17. The human phagocytic cell according to any one of embodiments 13 to 16, wherein the deletions are from 100 base pairs to 10000 base pairs each.
18. The human phagocytic cell according to any one of embodiments 13 to 17, wherein the deletions are from 500 base pairs to 3000 base pairs, such as from 600 base pairs to 1500 base pairs.
19. The human phagocytic cell according to any one of embodiments 13 to 18, wherein the MAFB deletions are within the region on human chromosome 22 from 40685000 to 40690000.
20. The human phagocytic cell according to embodiment 19, wherein the MAFB deletions are within the region on human chromosome 22 from 40685200 to 40689800, for example from 40685400 to 40689600, such as from 40685600 to 40689400 and in particular from 40685700 to 40689300.
21. The human phagocytic cell according to any one of embodiments 13 to 20, wherein the MAF deletions are within the region on human chromosome 16 from 79593000 to 79602000.
22. The human phagocytic cell according to embodiment 21, wherein the MAF deletions are within the region on human chromosome 16 from 79593200 to 79601500, for example from 79593400 to 79601100, and in particular from 79593600 to 79600900.
23. The human phagocytic cell according to any one of embodiments 1 to 22, wherein the phagocytic cell is derived from an iPS cell, a monocyte or a macrophage, in particular wherein the human phagocytic cell is selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow, mobilized monoblasts, myeloid or CD34+ multipotent progenitors from adult blood; monocytes, monoblasts, myeloid or CD34+ progenitors from extramedullary hematopoiesis; alveolar macrophages; and adipose tissue macrophages.
24. The human phagocytic cell according to any one of embodiments 1 to 23, wherein the phagocytic cell is genetically modified at a further locus other than MAFB or MAF.
25. The human phagocytic cell according to any one of embodiments 1 to 24, wherein the phagocytic cell is deficient for one or several HLA-I genes (human lymphocyte antigen I) deficient, for example HLA-A, HLA-B and/or HLA-C.
26. The human phagocytic cell according to any one of embodiments 1 to 25, wherein the phagocytic cell is B2M deficient.
27. The human phagocytic cell according to embodiment 26, wherein the phagocytic cell expresses HLA-E, HLA-F and HLA-G.
28. The human phagocytic cell according to any one of embodiments 1 to 27, wherein the phagocytic cell comprises a chimeric antigen receptor.
29. A composition comprising a human phagocytic cell according to any one of embodiments 1 to 28.
30. The composition according to embodiment 29 comprising from 10⁷ to 10¹² human phagocytic cells according to any one of embodiments 1 to 28.
31. The composition of embodiment 29 which is a pharmaceutical composition.
32. The pharmaceutical composition of embodiment 31 comprising from 10⁷ to 10¹¹ human phagocytic cells according to any one of embodiments 1 to 28.
33. The human phagocytic cell according to any one of embodiments 1 to 28 and/or the composition according to any one of embodiments 29 to 32 for use as a medicament.
34. The human phagocytic cell according to any one of embodiments 1 to 23 and/or the composition according to any one of embodiments 29 to 32 for use in the treatment of a disease selected from the group consisting of a a cancer, an immune-deficiency, a chronic or an acute injury, such as central nervous system injury - for example spinal cord injury - acute injury such as ischemic stroke, hepatic injury or myocardial infarction, a wound, such as a chronic wound, a degenerative disease, an autoimmune disease, such as type 1 diabetes or Crohn's disease, rheumatoid arthritis or osteoarthritis, a chronic inflammatory disease, atherosclerosis, poly- and osteoarthritis, osteoporosis, an infectious disease (e.g. infections by virus, or bacteria), and a metabolic disease.
35. The human phagocytic cell for use in the treatment of an immune deficiency according to embodiment 34, wherein the immune deficiency is an acute or acquired immune deficiency.
36. The human phagocytic cell for use in the treatment of an immune deficiency according to embodiment 34, wherein the immune deficiency is a result of chemotherapy or of a viral infection.
37. The human phagocytic cell for use in the treatment of an infectious disease, wherein the infectious disease is an infection of a virus, in particular an infection of the lung by a coronavirus such as SARS-CoV-2.
38. The human phagocytic cell for use in the treatment of a chronic or acute injury according to embodiment 34, wherein the chronic or acute injury is an injury of a bone or a muscle.
39. The human phagocytic cell for use in the treatment of a muscle injury according to embodiment 38, wherein the muscle is a cardiac muscle.
40. The human phagocytic cell for use in the treatment of an acute injury according to embodiment 34, wherein the acute injury is an injury of the spinal cord.
41. The human phagocytic cell for use in the treatment of a wound according to embodiment 34, wherein the wound is a skin wound.
42. The human phagocytic cell for use in the treatment of a degenerative disease according to embodiment 34, wherein the degenerative disease is arthritis.
43. The human phagocytic cell for use in the treatment of an ischemic disease according to embodiment 34, wherein the ischemic disease is ischemic heart disease.
44. The human phagocytic cell for use in the treatment of a metabolic disease according to embodiment 34, wherein the metabolic disease is diabetes.
45. The human phagocytic cell for use in the treatment of cancer according to embodiment 34, wherein the cancer is lung cancer.
46. The human phagocytic cell according to any one of embodiments 24 to 28 and/or the composition according to any one of embodiments 29 to 32 for use in the treatment of cancer.
47. A method for treating a human subject affected with a disease associated with a deficiency in human phagocytic cell function, which method comprises administering to said human subject a human phagocytic cell according to any one of embodiments 1 to 28 and/or the composition according to any one of embodiments 29 to 32.
48. A method for generating a human phagocytic cell according to any one of embodiments 1 to 23, the method comprising the step of
   a) effecting two double strand breaks each a1) within the region on human chromosome 22 from 40685000 to 40690000 and a2) within the region on human chromosome 16 from 79593000 to 79602000, in a human phagocytic cell; and
   b) selecting cells with deletions in both alleles of MAFB and in both alleles of MAF.
49. The method of embodiment 48, further comprising the step c) of selecting for proliferating cells.
50. The method according to any one of embodiments 48 or 49, wherein the double strand breaks in step a) are effected within the region on human chromosome 22 from 40685700 to 40689300 and within the region on human chromosome 16 from 79593600 to 79600900.
51. The method according to any one of embodiments 48 to 50 wherein the double strand breaks are effected by site-specific endonucleases or recombinases.
52. The method according to embodiment 51, wherein the site-specific endonucleases are Cas-9 in combination with at least four guide RNAs, wherein at least two of the at least four guide RNAs are directed at the MAFB locus on chromosome 22 and at least two of the at least four guide RNAs are directed at the MAF locus on chromosome 16.
53. The method according to any one of embodiments 48 to 52, wherein the human phagocytic cell used as the target cell for step a) is an iPS cell, a monocyte or a macrophage, in particular wherein the human phagocytic cell is selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow, mobilized monoblasts, myeloid or CD34+ multipotent progenitors from adult blood; monocytes, monoblasts, myeloid or CD34+ progenitors from extramedullary hematopoiesis; alveolar macrophages; and adipose tissue macrophages.
54. The method of embodiment 53, wherein the human phagocytic cell used as the target cell for step a) is an induced pluripotent stem cell.
55. The method of embodiment 54, wherein the induced pluripotent stem cell is an iPS cell.
56. The method according to any one of embodiments 48 to 55 further comprising the step d) of culturing the human phagocytic cells in a medium comprising M-CSF.
57. A method for generating a human phagocytic cell according to any one of embodiments 24 to 28, the method comprising genetically modifying a human phagocytic cell according to any one of embodiments 1 to 23 at a locus other than MAFB or MAF.
58. The method according to embodiment 57, wherein the genetic modification is deletion of one or several HLA-I genes (human lymphocyte antigen I), for example HLA-A, HLA-B and/or HLA-C.
59. The method according to embodiment 57, wherein the genetic modification is deletion of B2M.
60. The method according to embodiment 57, wherein the genetic modification is the introduction of a DNA encoding a chimeric antigen receptor.
61. A method for generating a human phagocytic cell according to any one of embodiments 24 to 28, wherein the starting cell for the method according to embodiments 47 to 56 is a phagocytic cell which is genetically modified at a locus other than MAFB or MAF.
62. A collection of human phagocytic cells, wherein the cells are non-tumorigenic, and wherein the number of cells increases at least 4-fold ex vivo after 8 days under suitable cultivation conditions.
63. The collection of human phagocytic cells of embodiment 62, wherein the number of cells is at least 1000.
64. The collection of human phagocytic cells according to any one of embodiments 62 to 63, wherein the number of cells is at least 100000, such as at least 1000000.
65. The collection of human phagocytic cells according to any one of embodiments 62 to 64, wherein the number of cells increases at least 10-fold under suitable cultivation conditions.
66. The collection of human phagocytic cells according to any one of embodiments 62 to 65, wherein the number of cells increases at least 20-fold under suitable cultivation conditions.
67. The collection of human phagocytic cells according to any one of embodiments 62 to 66, wherein the phagocytic cell is a professional phagocytic cell.
68. The collection of human phagocytic cells according to any one of embodiments 62 to 67, wherein the phagocytic cell is a myeloid cell.
69. The collection of human phagocytic cells according to any one of embodiments 62 to 68, wherein the phagocytic cell is a macrophage, monocyte or dendritic cell.
70. The collection of human phagocytic cells according to any one of embodiments 62 to 69, wherein the phagocytic cell is characterized by expression of a surface protein selected from the group consisting of CD45, CD64, CD11b, CD33, CD206 and CD14.
71. The collection of human phagocytic cells according to any one of embodiments 62 to 70, wherein the phagocytic cell is characterized in that the protein CD34 is not detectable on the surface of the phagocytic cell.
72. The collection of human phagocytic cells according to any one of embodiments 62 to 71, wherein the phagocytic cell is characterized by its capacity to perform phagocytosis, by its capacity to produce reactive oxygen species upon activation, by protease activity in its lysosomes, and/or by its capacity to take up lipids.
73. The collection of human phagocytic cells according to any one of embodiments 62 to 72, wherein the phagocytic cell is a large vacuolated cell and/or a cell with adherent morphology and/or a cell featuring lamello- and/or filopodia.
74. The collection of human phagocytic cells according to any one of embodiments 62 to 73, wherein the phagocytic cell has 46 chromosomes.
75. The collection of human phagocytic cells according to embodiment 74, wherein the phagocytic cell does not contain a chromosome with a chromosome rearrangement.
76. The collection of human phagocytic cells according to any one of embodiments 62 to 75, wherein the phagocytic cell is differentiated.
77. The collection of human phagocytic cells according to any one of embodiments 62 to 76, wherein the phagocytic cell does not form a tumor upon intratracheal transplantation into huPAP mice.
78. The collection of human phagocytic cells according to any one of embodiments 62 to 77, wherein the phagocytic cell is characterized in that both alleles of MAFB and both alleles of MAF have been rendered nonfunctional by deletions of at least 50 base pairs.
79. The collection of human phagocytic cells according to embodiment 78, wherein all deletions comprise exonic DNA.
80. The collection of human phagocytic cells according to any one of embodiments 78 to 79, wherein the deletions are at least 100 base pairs each.
81. The collection of human phagocytic cells according to any one of embodiments 78 to 80, wherein the deletions are at least 250 base pairs each.
82. The collection of human phagocytic cells according to any one of embodiments 78 to 81, wherein the deletions are from 100 base pairs to 10000 base pairs each.
83. The collection of human phagocytic cells according to any one of embodiments 78 to 83, wherein the deletions are from 500 base pairs to 3000 base pairs.
84. The collection of human phagocytic cells according to any one of embodiments 78 to 84, wherein the MAFB deletions are within the region on human chromosome 22 from 40685000 to 40690000.
85. The collection of human phagocytic cells according to embodiment 84, wherein the MAFB deletions are within the region on human chromosome 22 from 40685700 to 40689300.
86. The collection of human phagocytic cells according to any one of embodiments 78 to 85, wherein the MAF deletions are within the region on human chromosome 16 from 79593000 to 79602000.
87. The collection of human phagocytic cells according to embodiment 86, wherein the MAF deletions are within the region on human chromosome 16 from 79593600 to 79600900.
88. The collection of human phagocytic cells according to any one of embodiments 62 to 87, wherein the phagocytic cell is derived from an iPS cell, a monocyte or a macrophage, in particular wherein the human phagocytic cell is selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow, mobilized monoblasts, myeloid or CD34+ multipotent progenitors from adult blood; monocytes, monoblasts, myeloid or CD34+ progenitors from extramedullary hematopoiesis; alveolar macrophages; and adipose tissue macrophages.
89. The collection of human phagocytic cells according to any one of embodiments 62 to 88, wherein the phagocytic cell is genetically modified at a locus other than MAFB or MAF.
90. The collection of human phagocytic cells according to any one of embodiments 62 to 89, wherein the phagocytic cell is deficient for one or several HLA-I genes (human lymphocyte antigen I) deficient, for example HLA-A, HLA-B and/or HLA-C.
91. The collection of human phagocytic cells according to any one of embodiments 62 to 90, wherein the phagocytic cell is B2M deficient.
92. The collection of human phagocytic cells according to embodiment 91, wherein the phagocytic cell expresses HLA-E, HLA-F and HLA-G.
93. The collection of human phagocytic cells according to any one of embodiments 62 to 92, wherein the phagocytic cell comprises a chimeric antigen receptor.
94. A human phagocytic cell, wherein both alleles of MAFB and both alleles of MAF have been rendered nonfunctional by deletions of at least 50 base pairs.
95. The human phagocytic cell according to embodiment 94, wherein all deletions comprise exonic DNA.
96. The human phagocytic cell according to any one of embodiments 94 to 95, wherein the deletions are at least 100 base pairs each.
97. The human phagocytic cell according to any one of embodiments 94 to 96, wherein the deletions are at least 250 base pairs each.
98. The human phagocytic cell according to any one of embodiments 94 to 97, wherein the deletions are from 100 base pairs to 10000 base pairs each.
99. The human phagocytic cell according to any one of embodiments 94 to 98, wherein the deletions are from 500 base pairs to 3000 base pairs.
100. The human phagocytic cell according to any one of embodiments 94 to 99, wherein the MAFB deletions are within the region on human chromosome 22 from 40685000 to 40690000.
101. The human phagocytic cell according to embodiment 100, wherein the MAFB deletions are within the region on human chromosome 22 from 40685700 to 40689300.
102. The human phagocytic cell according to any one of embodiments 94 to 101, wherein the MAF deletions are within the region on human chromosome 16 from 79593000 to 79602000.
103. The human phagocytic cell according to embodiment 102, wherein the MAF deletions are within the region on human chromosome 16 from 79593600 to 79600900.
104. The human phagocytic cell according to any one of embodiments 94 to 103, wherein the phagocytic cell is derived from an iPS cell, a monocyte or a macrophage, in particular wherein the human phagocytic cell is selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow, mobilized monoblasts, myeloid or CD34+ multipotent progenitors from adult blood; monocytes, monoblasts, myeloid or CD34+ progenitors from extramedullary hematopoiesis; alveolar macrophages; and adipose tissue macrophages.
105. The human phagocytic cell according to any one of embodiments 94 to 104, wherein the phagocytic cell is characterized by expression of a surface protein selected from the group consisting of CD45, CD64, CD11b, CD33, CD206 and CD14.
106. The human phagocytic cell according to any one of embodiments 94 to 105, wherein the phagocytic cell is characterized in that the protein CD34 is not detectable on the surface of the phagocytic cell.
107. The human phagocytic cell according to any one of embodiments 94 to 106, wherein the phagocytic cell is characterized by its capacity to perform phagocytosis, by its capacity to produce reactive oxygen species upon activation, by protease activity in its lysosomes, and/or by its capacity to take up lipids.
108. The human phagocytic cell according to any one of embodiments 94 to 107, wherein the phagocytic cell is a large vacuolated cell and/or a cell with adherent morphology and/or a cell featuring lamello- and/or filopodia.
109. The human phagocytic cell according to any one of embodiments 94 to 108, wherein the phagocytic cell has 46 chromosomes.
110. The human phagocytic cell according to embodiment 109, wherein the phagocytic cell does not contain a chromosome with a chromosome rearrangement.
111. The human phagocytic cell according to any one of embodiments 94 to 110, wherein the phagocytic cell is non-tumorigenic.
112. The human phagocytic cell according to any one of embodiments 94 to 111, wherein the phagocytic cell does not form a tumor upon transplantation into huPAP mice.
113. The human phagocytic cell according to any one of embodiments 94 to 112, wherein the phagocytic cell is a professional phagocytic cell.
114. The human phagocytic cell according to any one of embodiments 94 to 113, wherein the phagocytic cell is a myeloid cell.
115. The human phagocytic cell according to embodiment 114, wherein the phagocytic cell is a macrophage, monocyte or dendritic cell.
116. The human phagocytic cell according to any one of embodiments 94 to 115, wherein the phagocytic cell is genetically modified at a locus other than MAFB or MAF.
117. The human phagocytic cell according to any one of embodiments 94 to 116, wherein the phagocytic cell is deficient for one or several HLA-I genes (human lymphocyte antigen I) deficient, for example HLA-A, HLA-B and/or HLA-C.
118. The human phagocytic cell according to any one of embodiments 94 to 117, wherein the phagocytic cell is B2M deficient.
119. The human phagocytic cell according to embodiment 118, wherein the phagocytic cell expresses HLA-E, HLA-F and HLA-G.
120. The human phagocytic cell according to any one of embodiments 94 to 119, wherein the phagocytic cell comprises a chimeric antigen receptor.
121. A composition comprising a human phagocytic cell according to any one of embodiments 94 to 120.
122. The composition according to embodiment 121 comprising from 10⁷ to 10¹² human phagocytic cells according to any one of embodiments 1 to 28.
123. The composition of embodiment 122 which is a pharmaceutical composition.
124. The pharmaceutical composition of embodiment 123 comprising from 10⁷ to 10¹¹ human phagocytic cells according to any one of embodiments 1 to 28.
125. The human phagocytic cell according to any one of embodiments 94 to 120 and/or the composition according to any one of embodiments 121 to 124 for use as a medicament.
126. The human phagocytic cell according to any one of embodiments 94 to 120 and/or the composition according to any one of embodiments 121 to 124 for use in the treatment of a disease selected from the group consisting of a cancer, an immune-deficiency, a chronic or an acute injury, such as central nervous system injury - for example spinal cord injury - acute injury such as ischemic stroke, hepatic injury or myocardial infarction, a wound, such as a chronic wound, a degenerative disease, an autoimmune disease, such as type 1 diabetes and Crohn's disease, rheumatoid arthritis or osteoarthritis, a chronic inflammatory disease, atherosclerosis, poly- and osteoarthritis, osteoporosis, an infectious disease (e.g. infections by virus, or bacteria), and a metabolic disease.
127. A composition comprising a collection of cells obtainable by cultivating a collection of cells according to any one of embodiments 62 to 93 under conditions of cultivation suitable for an increase in cell numbers.
128. The composition according to embodiment 127 for use as a medicament.
129. The composition according to embodiment 127 for use in the treatment of a disease selected from the group consisting of a cancer, an immune-deficiency, a chronic or an acute injury, such as central nervous system injury - for example spinal cord injury - acute injury such as ischemic stroke, hepatic injury or myocardial infarction, a wound, such as a chronic wound, a degenerative disease, an autoimmune disease, such as type 1 diabetes and Crohn's disease, rheumatoid arthritis or osteoarthritis, a chronic inflammatory disease, atherosclerosis, poly- and osteoarthritis, osteoporosis, an infectious disease (e.g. infections by virus, or bacteria), and a metabolic disease.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques are applicable to the production of the polynucleotides and polypeptides of the invention, and, as such, may be considered in making and practicing the invention.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### EXAMPLES

Methods for the generation of human induced pluripotent stem cells are well known in the art. A human iPS cell line was prepared from dermal fibroblasts obtained from the prepuce of the penis of a healthy human caucasian neonate male. Reprogramming was with non-integrating Sendai virus containing POU5F1, SOX2, KLF4 and MYC (Fusaki N., et al. (2009)). Virus screening for HIV1, HIV2, hepatitis virus B and C and mycoplasm was negative. Karyotypic showed a normal 46,X,Y karyotype. Into this cell line a doxycycline-inducible Cas9-expression cassette was introduced into the AAVS1 locus through TALEN-mediated gene targeting, essentially as described in Gonzalez et al. (2014), providing cell line BIHi001-A-1.

### Example 1

### Generation of double knock-out (DKO) cells from iPS cells

### a) iPSC culture

iPSCs were cultured under feeder-free conditions in StemFlex Medium (Thermo Fisher, #A3349401) on vitronectin-coated (Thermo Fisher, # A14700) tissue-culture plates at 37°C, 5% CO2 under normoxic conditions. Cells were passaged with ReLeSR (Stem Cell Technologies, #05872) when reaching 60-80% confluency and maintained in the presence of 10 µM of the ROCK inhibitor Y-27632 (biomol, #Cay1000SS83) on the day after passaging. Single-cell suspensions of iPSC were obtained by incubation with Accutase (Merck Millipore, #SF006).

### b) Genome editing

We used the CRISPR/Cas9 system to delete the coding sequence of MAF (Gene ID: 4094, 373 amino acids) and MAFB (Gene ID: 9935, 323 amino acids) in human cells. Towards this end, we transfected a healthy-donor-derived iPSC line harbouring a doxycycline-inducible Cas9 expression cassette integrated into the AAVS1 locus (BIHi001-A-1, https://hpscreg.eu/cell-line/BIHi001-A-1; also called iBCRT Cas9v1-3G-Kl.16) with sgRNA-expressing plasmids targeting MAF and MAFB (pU6-(Bbsl)sgRNA_CAG-venus-bpA, Addgene ID 86985, https://www.addgene.org/86985/) according to a published protocol (Yumlu, (2017)).

To delete the complete CDS, we designed 2 sgRNAs per gene to target a sequence in the 5'UTR at least 20 nt upstream of the start codon and in the 3'UTR at least 20 nt downstream of the stop codon of each gene using the CrispRGold program for sequence design (https://crisprgold.mdcberlin.de/ Chu et al. (2016)). The 2 sgRNAs for each gene were expressed together from the sgRNA-expression vector pU6-(Bbsl)sgRNA_CAG-venus-bpA, which also encodes a fluorescent reporter gene (Venus, derived from YFP) for positive selection of transfected cells by FACS.

**Table 1: sgRNA and protospacer sequences for CRISPR/Cas9-mediated deletion of MAF and MAFB CDS. The position of the target-specific, 20-nucleotide long protospacer is marked by "N" within the sgRNA sequence. The genomic target sequences of the sgRNAs expressed from pU6-(Bbsl)sgRNA_CAG-venus-bpA with the indicated protospacer sequences are located in the 5'UTR and 3'UTR, respectively, of MAF and MAFB. UTR, untranslated region.**

| **sgRNA** | | |
|---|---|---|
| NNNNNNNNNNNNNNNNNNNNGTTTTAGAGCTAGAAATAGCAAGTTAAAATAAGGCTAG TCCGTTATCAACTTGAAAAAGTGGCACCGAGTCGGTGCTTT | | |
| | | |

| **Locus** | **Protospacer sequence for 5'UTR** | **Protospacer sequence for 3'UTR** |
|---|---|---|
| MAF | ATCTGGCGGAGCGGCGGCGG | ACCCTGATAAGTGCTCCGCG |
| MAFB | CGGCCGCAAAGTTTCCCGGG | TGACCTGTTTGACTTGAGCG |

SEQ ID No.1 (MAF-5'UTR):
5'ATCTGGCGGAGCGGCGGCGGGTTTTAGAGCTAGAAATAGCAAGTTAAAATAAGGCTAGTCCGTT
ATCAACTTGAAAAAGTGGCACCGAGTCGGTGCTTT-3'

SEQ ID No.2 (MAF-3'UTR):
5'ACCCTGATAAGTGCTCCGCGGTTTTAGAGCTAGAAATAGCAAGTTAAAATAAGGCTAGTCCGTT
ATCAACTTGAAAAAGTGGCACCGAGTCGGTGCTTT-3'

SEQ ID No.3 (MAFB-5’UTR):
5’CGGCCGCAAAGTTTCCCGGGGTTTTAGAGCTAGAAATAGCAAGTTAAAATAAGGCTAGTCCGTT
ATCAACTTGAAAAAGTGGCACCGAGTCGGTGCTTT-3’

SEQ ID No.4 (MAFB-3'UTR):
5'TGACCTGTTTGACTTGAGCGGTTTTAGAGCTAGAAATAGCAAGTTAAAATAAGGCTAGTCCGTT
ATCAACTTGAAAAAGTGGCACCGAGTCGGTGCTTT-3'

Using Lipofectamine 3000 (Thermo Fisher, #L3000001) we transfected BIHi001-A-1 with sgRNA expression vectors together with pCAG-mTrex2-bpA (Addgene ID 86984, https://www.addgene.org/86984/) to enhance the propensity of indel formation. Control cells, labelled with "WT" or "wild-type" throughout the text, were treated the same way except for using the pU6-(Bbsl)sgRNA_CAG-venus-bpA without inserting any protospacer sequence. Transfected BIHi001-A-1 cells were treated with 1 µg/ml doxycycline hyclate (Sigma-Aldrich, #D9891) to induce Cas9 expression, followed by FACS-isolation of cells expressing the Venus reporter gene from the sgRNA-carrying vector. Sorted cells were seeded at low density to allow the isolation of single-cell-derived colonies. In the first round of transfection, sgRNAs targeting MAF were used and colonies were screened for full-length deletion of the MAF CDS using PCR and Sanger sequencing of the edited locus (Figure 1). Cells were cultured under conditions for iPS cells, as described above.

MAF KO iPSC were subjected to a second round of transfection using MAFB-targeting sgRNAs, followed by PCR and sequencing analysis of the obtained colonies. Three MAF/MAFB DKO (MAF-DKO) iPSC clones were isolated (clone 1, 2, 3). The genomic target region and the edited MAF and MAFB loci are shown in Figure 2.

### Example 2

### Characterization of MAF/MAFB DKO iPS cells

The 3 isolated MAF-DKO iPSC clones were analyzed by karyotyping. Karyotyping was performed by conventional cytogenetic analysis (G-banding). Briefly, cells were blocked with 100 ng/ml Colchemid for 2,5 h at 37°C, 5% CO2, enlarged using 0.075 M KCl and fixed with 3:1 methanol:glacial acetic acid. Metaphase chromosomes were spread on coverslips and stained with Giemsa staining. 20 metaphase spreads per sample were analyzed.

All clones had a normal appearance and karyotype (Figure 3).

### Example 3

### Directed differentiation of iPSC into macrophages

Directed differentiation of iPSC into macrophages was based on previously published protocols (Buchrieser 2017). Embryoid bodies (EBs) were formed by seeding a single-cell suspension of wild-type or MAF-DKO iPSC at a density of 12,500 cells/well into an ultra-low attachment U-shaped-bottom 96-well plate (Nunclon Sphera, Thermo Fisher, #174925), resuspended in EB medium consisting of StemFlex Medium (Thermo Fisher, #A3349401) supplemented with 50 ng/ml BMP-4 (R&D Systems, #314-BP), 20 ng/ml SCF (R&D systems, 255-SC), 50 ng/ml VEGF (Peprotech, AF-100-20A) and 10 µM Y-27632. The 96-well plate was centrifuged for 3 minutes at 100 g to collect the cells at the bottom, and placed at 37°C, 5% CO2 for 4 days. After 1 and 2 days, half of the EB medium was replaced with freshly prepared EB medium.

Wild-type and MAF-DKO EBs were indistinguishable by size or morphology (Figure 4).

After 4 days, EBs were selected manually, resuspended in EB differentiation medium consisting of X-VIVO 15 (Lonza, #BE02-060F) supplemented with 2 mM GlutaMAX (Thermo Fisher, #35050038), 0.055 mM 2-mercaptoethanol (Sigma-Aldrich, #M6250), 100 ng/ml human M-CSF (Thermo Fisher, #PHC9504), 25 ng/ml human IL-3 (R&D systems, #203-IL), and seeded into ultra-low attachment 6-well tissue-culture plates (8 EBs/well) or 90 mm tissue-culture dishes (24 EBs/dish; Nunclon Sphera, Thermo Fisher, #174932 and #174945). Two thirds of the culture medium were exchanged with fresh differentiation medium every 5 days. Production of monocytes/macrophages from EBs started around day 15 post EB seeding, and suspension cells were harvested, counted and used for immunophenotyping by flow cytometry or EdU incorporation assays.

For further experiments the cells harvested from the supernatant of the EB cultures were replated for final macrophage differentiation in RPMI supplemented with 10% FBS (PAA-GE Healthcare, A15-101), supplemented with 100 units/ml penicillin, 100 ug/ml streptomycin (Thermo Fisher, #15140122), 2 mM GlutaMAX (Thermo Fisher, #35050038), 1 mM sodium pyruvate (Thermo Fisher, #11360-039), 50 ng/ml M-CSF (Thermo Fisher, #PHC9504), seeded into ultra-low attachment 12-well tissue-culture plates (Nunclon Sphera, Thermo Fisher #174931, #174932) and 50 ng/ml GM-CSF (Peprotech, #300-03); 37°C, 5% CO2. Cells were counted manually using a Neubauer hemocytometer or automatically with a CASY cell counter (OMNI Life Science).

Deletion of MAF and MAFB had no effect on myeloid differentiation capacity, and, similar to wild-type EBs, MAF-DKO EBs started releasing monocytes/macrophages into the EB differentiation medium around day 15 post EB seeding. MAF-DKO suspension cells were viable, and after plating in differentiation medium containing 50 ng/ml M-CSF and GM-CSF, both wild-type and MAF-DKO macrophages phagocytosed beads, produced ROS, and stained positive for lysosomes and cathepsin activity, indicative of a mature macrophage phenotype (Figure 5).

### Example 4

### Characterization of MAF/MAFB DKO cells after final macrophage-differentiation

For flow cytometry, replated cells obtained from EB-differentiation cultures on day 15 as described in example 3 and grown for 7 days under final macrophage differentiation conditions were blocked with FcR Blocking Reagent (Miltenyi, # 130-059-901) for 15 minutes at 4°C, washed and stained with antibodies according to Table 2 for 30 minutes on 4°C. Cells were recorded on an LSRFortessa (BD) cytometer and analysed with FlowJo (BD).

**Table 2: Antibodies used for immunophenotyping.**

| **Target** | **Company** | **Cat. Number** | **Concentration** |
|---|---|---|---|
| CD45-APC/Cy7 | Biolegend | 304042 | 1/200 |
| CD11b-BV421 | BD Pharmingen | 557657 | 1/200 |
| CD33-BV785 | Biolegend | 303427 | 1/200 |
| CD14-APC | Miltenyi | 130-091-243 | 1/200 |
| CD64-PE | Biolegend | 305007 | 1/200 |
| CD206-PE/Cy7 | eBioscience | 25-2069-42 | 1/200 |

We did not observe major differences between wild-type and MAF-DKO macrophages regarding morphology or immunophenotype as assessed by flow cytometry (Figure 6) or histological staining.

MAF-DKO macrophages were tested for MAF and MAFB protein and mRNA expression.

RNA was extracted from wild-type and MAF-DKO cells using the RNeasy Mini kit (Qiagen, #74104), followed by cDNA synthesis using the High Capacity Reverse Transcription kit (Applied Biosystems, #4368814). qPCR was performed using the TB Green Premix Ex Taq (Tli Rnase plus, Takara, #RR420L). qPCR products were run on a 2% agarose gel. For Western Blotting, wild-type and MAF-DKO protein was extracted by lysing cells in Laemmli buffer. Lysates were cleared with QIAshredder (Qiagen, #79656) denatured and loaded on a polyacrylamide gel for electrophoresis (SDS-PAGE), followed by semi-dry blotting. Blots were blocked with 5% milk in TBST and incubated with anti-MAFB (Sigma, #HPA005653) or anti-MAF (Sigma, #HPA028289) antibodies, then with a secondary antibody, anti-rabbit IgG-HRP, followed by incubation with ECL^{™} Prime Western Blot detection reagent (GE Healthcare Amersham^{™}, #RPN2232)
Importantly, wild-type iPSC-derived macrophages did express both MAF and MAFB mRNA and protein, respectively, whereas MAF-DKO macrophages were negative for MAF and MAFB expression on mRNA and protein level (Figure 7).

### Example 5

### MAF-DKO macrophages are functional and treat pulmonary alveolar proteinosis in a humanized mouse model

To test the in vivo functionality of MAF-DKO macrophages, we selected a mouse strain called huPAP, which is an animal model for pulmonary alveolar proteinosis (PAP), a human lung disease (Official name: C;129S4-Rag2tm1.1Flv Csf2/Il3tm1.1(CSF2,IL3)Flv Il2rgtm1.1Flv/J; JAX # 014595). The huPAP strain is an immuno-deficient mouse line designed for transplantation of human cells. Due to the lack of murine GM-CSF expression, the lungs are devoid of alveolar macrophages, hence the mice show signs of alveolar proteinosis, e.g., high protein content in the fluid obtained through bronchoalveolar lavage (BAL), resulting in higher turbidity. Instead of murine GM-CSF, huPAP express human GM-CSF (and IL-3), allowing not only the reconstitution of alveolar macrophages with transplanted human cells but also the rescue of the alveolar proteinosis phenotype. Thus, this model is a useful tool to study in vivo functionality of human macrophages.

We transplanted equal numbers (between 2x10⁶ and 4x10⁶ per transplantation) of either wild-type or MAF-DKO macrophages (harvested from EB-cultures between day 15 and day 25 after resuspension in EB differentiation medium) intratracheally into huPAP mice (Figure 8) together with 1µg M-CSF per animal to check for both cellular engraftment and rescue of PAP. Control mice received an equal volume of PBS (cell resuspension buffer).

HuPAP recipients of both wild-type of MAF-DKO macrophages recovered quickly after each transplantation and did not demonstrate abnormal behaviour compared to PBS-treated or non-treated animals (data not shown). All animals were transplanted 4 times with wild-type or MAF-DKO macrophages, each transplantation separated by 1 week. The animals were analysed four weeks after the last transplantation.

On macroscopic examination, we did not observe tumours neither within the lung tissue nor in the other organs, consistent with the fact that MAF-DKO macrophages maintained a normal karyotype without obvious chromosomal aberrations (Figure 3).

We found that MAF-DKO macrophages showed better engraftment into huPAP mice than wild-type macrophages (Figure 9) and improved rescue of the PAP phenotype, demonstrated by a reduction in turbidity, protein concentration and levels of SP-D in the bronchoalveolar lavage fluid, that are slightly better than the respective rescue by wild-type macrophages (Figure 10). Furthermore, wild-type and MAF-DKO macrophages isolated from engrafted mice 4 weeks after the last treatment phagocytosed beads, produced ROS, stained positive for lysosomes and cathepsin activity, and were also able to engulf lipids. (Figure 11, Figure 12).

### Example 6

### Increased proliferation of MAF-DKO cells

Both wild-type and MAF-DKO EBs started producing monocytes/macrophages between day 10-15 post EB-resuspension in EB differentiation medium (first harvest and cell count was on day 15). However, when we quantified the numbers of cells released into the EB supernatant, we found that differentiation of MAF-DKO EBs resulted in largely increased production yields of macrophages (Figure13). The cumulative number of macrophages released by MAF-DKO EBs after 30 days of EB-differentiation was at least 6.6-fold (16.58±1.29) higher than the yield of wild-type EBs (2.51±0.18) (figure 13b).

Furthermore, we observed that the MAF-DKO macrophages harvested from the supernatant of the EB-differentiation proliferated further in the final macrophage differentiation phase. When the cells harvested from the supernatant of the EB-differentiation cultures were replated in the final macrophage differentiation medium (RPMI plus supplements; example 3) MAF-DKO cell numbers increased by a factor of 25 by day 12 of replating, while wild-type cell numbers increased only by a factor of 3 (figure 13d). Together, the theoretical numbers of obtained MAF-DKO macrophages per input iPSC corresponded to about 1000 and thus was about 100 times higher than the most efficient protocol published so far, and 67 times higher than the numbers of wild-type cells produced in our protocol (figure 15).

Consistent with this, MAF-DKO macrophages produced a significantly higher number of colonies compared to wild-type macrophages in a semi-solid methylcellulose-based colony assay.

To assess the proportion of cells in S-phase, we pulse-labeled wild-type and MAF-DKO macrophages for 4 h with EdU and analysed the percentage of EdU incorporation by flow cytometry. We found that MAF-DKO macrophages had an 8-fold increase in EdU-positive cells compared to wild-type macrophages (17.6% and 2.2% EdU-positive cells, respectively, Figure 14).

In conclusion, these data demonstrate that deletion of MAF and MAFB in human macrophages results in extended proliferative potential without causing tumorigenicity and without losing macrophage characteristics such as phagocytosis in vitro and in vivo.

## Claims

1. An ex vivo proliferating human phagocytic cell, wherein the cell is non-tumorigenic.

2. The human phagocytic cell of claim 1, wherein both alleles of MAFB and both alleles of MAF have been rendered nonfunctional by deletions of at least 50 base pairs.

3. The human phagocytic cell according to any one of claims 1 to 2, wherein the phagocytic cell is a macrophage, monocyte or dendritic cell.

4. The human phagocytic cell according to any one of claims 1 to 3, wherein all deletions comprise exonic DNA.

5. The human phagocytic cell according to any one of claims 1 to 4, wherein the deletions are from 200 base pairs to 3000 base pairs.

6. The human phagocytic cell according to any one of claims 1 to 5, wherein the phagocytic cell is derived from an iPS cell, a monocyte or a macrophage, in particular wherein the human phagocytic cell is selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow, mobilized monoblasts, myeloid or CD34+ multipotent progenitors from adult blood; monocytes, monoblasts, myeloid or CD34+ progenitors from extramedullary hematopoiesis; alveolar macrophages; and adipose tissue macrophages.

7. A collection of human phagocytic cells, wherein the cells are non-tumorigenic, and wherein the number of cells increases at least 4-fold under suitable cultivation conditions.

8. The collection of human phagocytic cells according to claim 7, wherein the number of cells is at least 1000000.

9. The collection of human phagocytic cells according to any one of claims 7 to 8, wherein the number of cells increases at least 10-fold under suitable cultivation conditions.

10. The collection of human phagocytic cells according to any one of claims 7 to 9, wherein the collection of human phagocytic cells comprises human phagocytic cells according to any one of claims 2 to 6.

11. The human phagocytic cell according to any one of claims 1 to 6 or the collection of human phagocytic cells according to any one of claims 7 to 10 for use as a medicament.

12. The human phagocytic cell according to any one of claims 1 to 6 or the collection of human phagocytic cells according to any one of claims 7 to 10 for use in the treatment of a disease selected from the group consisting of a cancer, an immune-deficiency, a chronic or an acute injury, a wound, a degenerative disease, an autoimmune disease, an acute or chronic inflammatory disease, atherosclerosis, rheumatoid arthritis, osteoporosis, an infectious disease (e.g. infections by virus, or bacteria), and a metabolic disease.
